Europäisches Patentamt

European Patent Office

Office européen des brevets

(11) Publication number: **0 194 006 B1**

# EUROPEAN PATENT SPECIFICATION

(45) Date of publication of patent specification: **29.04.92**

(51) Int. Cl.5: **C07K 15/26**, C12N 15/00, C12P 21/02, A61K 37/66

(21) Application number: **86300304.2**

(22) Date of filing: **17.01.86**

(54) **Analogous interferon polypeptides, process for their preparation and pharmaceutical compositions containing them.**

(30) Priority: **01.02.85 GB 8502605**
**25.06.85 US 748558**

(43) Date of publication of application:
**10.09.86 Bulletin 86/37**

(45) Publication of the grant of the patent:
**29.04.92 Bulletin 92/18**

(84) Designated Contracting States:
**AT BE CH DE FR GB IT LI LU NL SE**

(56) References cited:
**EP-A- 0 032 134          EP-A- 0 051 873**
**EP-A- 0 062 971          EP-A- 0 072 541**
**EP-A- 0 076 489          EP-A- 0 089 692**
**EP-A- 0 100 561          WO-A-83/02457**
**WO-A-83/02458          WO-A-83/02459**
**WO-A-83/02460          WO-A-83/04053**
**WO-A-84/00776**

**SCIENCE, vol. 212, 5th June 1981, pages 1159-1162, AAAS; R. LAWN et al.: "DNA sequence of two closely linked human leukocyte interferon genes"**

(73) Proprietor: **IMPERIAL CHEMICAL INDUSTRIES PLC**
**Imperial Chemical House, Millbank**
**London SW1P 3JF(GB)**

(72) Inventor: **Camble, Roger**
**8 Yew Tree Close**
**Macclesfield Cheshire(GB)**
Inventor: **Edge, Michael Derek**
**6 Tudor Way**
**Congleton Cheshire(GB)**

(74) Representative: **Mack, John Richard et al**
**Imperial Chemical Industries PLC Legal Department: Patents PO Box 6 Bessemer Road Welwyn Garden City Hertfordshire AL7 1HD(GB)**

**Description**

The present invention relates to polypeptides, particularly interferon analogues, such as analogues of human interferon alpha$_2$, processes for their preparation and pharmaceutical compositions containing them as well as to the genetic modification of microorganisms to express the said analogues, the microorganisms thereby obtained, the genetic material employed in the modification and vectors therefor. The human interferon alpha$_2$ (hereinafter also referred to as IFN-alpha$_2$) analogues of the present invention possess interesting physiological activity.

Natural interferons are known compounds which are produced by a variety of living cells both in vivo and in vitro in tissue cultures in response to certain inducers, such as viral infection, immune stimulation and a range of chemical inducers. They are defined as proteins able to inhibit the replication of a variety of RNA and DNA viruses through cellular metabolic processes involving the synthesis of new RNA and protein (Committee on Interferon Nomenclature, 1980). Three main classes of natural interferons have been distinguished to date and are currently named alpha (IFN- $\alpha$), beta (IFN- $\beta$ ) and gamma (IFN- $\gamma$ ) on the basis of antigenic types. Interferons, for example, IFN-alpha possess antiviral, antiproliferative and immunomodulatory activity.

Information on the biological properties of interferons has come predominantly from studies of material produced by cell culture techniques. Recent results using recombinant DNA techniques have shown that human leukocyte interferon (Hu-IFN-alpha) is a family of at least 8 different molecules (Goeddel et al, Nature, 1981, 290, pages 20-26). Furthermore, Allen and Fantes (Nature, 1980, 287 pages 408-411) have demonstrated that microheterogeneities in interferon preparations from Namalwa cells can be ascribed to the presence of at least five homologous proteins with different amino acid sequences and hence to different structural genes. Despite these advances, very little is known about the distinct physiological properties of individual sub-species of Hu-IFN-alpha.

As stated above human leukocyte interferon is a family of a number of different molecules such as IFN-alpha$_1$ and IFN-alpha$_2$, the present invention being concerned inter alia with analogues or IFN-alpha$_2$.

The preparation of certain truncated analogues of IFN-alpha$_2$ has been described in the literature. Thus it is stated in Weck, P. K. et al. J. Cell. Biochem. Suppl. 6:104, 1982 that studies with hybrid leukocyte interferons indicate that both the N- and the C-terminal portions of the leukocyte interferons are involved in receptor binding. These studies involved certain hybrids and indicated that the C-terminal 15 amino acids clearly have significant effects on the activity of these interferons. Nevertheless a number of analogues of interferon alpha$_2$ have been prepared having a truncated C-terminus and for example, IFN-alpha$_2$ (1-160), IFN-alpha$_2$ (1-155) and IFN-alpha$_2$ (1-152) have all been reported to retain their antiviral activity, but show no adequate separation of antiviral from antiproliferative or NK-cell stimulating activities relative to IFN-alpha$_2$.

IFN-alpha$_2$ (4-160) and IFN-alpha$_2$ (4-165) have also been disclosed, but these truncated analogues also showed no significant separation of antiviral from antiproliferative or NK-cell stimulating activities relative to IFN-alpha$_2$. We have found, however, that IFN-alpha$_2$ (4-155) showed a consistent ten-fold increase over IFN-alpha$_2$ in the number of antiviral units required to produce a 50% inhibition of Daudi cell proliferation.

The literature also contains a number of references to the production of terminal hybrids of IFN-alpha$_2$ with IFN-alpha$_1$ or with IFN-alpha I. It will be understood that references herein to a terminal hybrid mean a hybrid of two interferon molecules in which the hybrid consists of two sections, the N-terminal section of the hybrid comprising the amino acids in the sequence of one interferon and the C-terminal section comprising the amino acids in the sequence of the other interferon. Thus IFN-alpha$_1$ (1-92)/alpha$_2$ (92-165), IFN-alpha$_1$ (1-62)/alpha$_2$ (62-165), IFN-alpha$_2$ (1-91)/alpha$_1$ (93-166) and IFN-alpha$_2$ (1-61)/alpha$_1$ (63-166) have all been described as possessing antiviral activity in Weck, P. K. et al (1981) "Antiviral activities of hybrids of two major human leukocyte interferons", Nucleic Acids Research 9, 6153-6166. IFN-alpha$_2$ (1-149)/alpha-I (151-166), IFN-alpha-I (1-150)/alpha$_2$ (150-165), IFN-alpha$_2$ (1-149)/alpha$_1$ (151-166) and IFN-alpha$_1$ (1-150)-/alpha$_2$ (150-165) have all been described as possessing antiviral activity in Franke, A. E. et al (1982). "Carboxyterminal region of hybrid leukocyte interferons affects antiviral specificity". DNA, 1, 223-230.

One internal $\alpha_1/\alpha_2$ hybrid (in which an IFN-alpha$_1$ segment is flanked by segments of interferon alpha$_2$) has been disclosed as possessing antiviral activity and this analogue is [Thr$^{68}$,Asp$^{79}$,Cys$^{85}$]IFN-alpha$_2$. This analogue is disclosed in Rehberg, E. et al (1982) "Specific molecular activities of recombinant and hybrid leukocyte interferons "Journal of Biological Chemistry, 257, 11497-11502.

The present invention is based on the discovery that novel polypeptides possessing at least certain of the properties of natural interferons, but in particular good antiviral activity, may be prepared by effecting one or more amino acid or sequence changes as hereinafter defined to the structure of IFN-alpha$_2$ or its

2

EP 0 194 006 B1

known analogues. Whilst the amino acid sequence for IFN-alpha$_2$ is known, it is not known, and it cannot be accurately predicted, what changes to the IFN-alpha$_2$ polypeptide may be made without losing antiviral activity. It is known that apparently small changes to polypeptide molecules made in one region of the polypeptide may destroy all biological activity whereas an improvement in biological activity may be obtained by an equally small change in a different region of the polypeptide.

The polypeptides of the present invention may also be used as biochemical tools, for example, after immobilization on an appropriate support they may be used for the enrichment and purification of interferon receptors or they may be useful in discriminating between families of monoclonal antibodies raised against native interferons.

It is to be understood that in this specification the abbreviations used for amino acids are standard abbreviations used in the peptide art (see Pure and Applied Chemistry, 1974, 40, 317-331, and Neuropeptides, 1981, 1, 231-235). Thus for example the following amino acids are abbreviated as indicated:-

| Alanine | Ala | Leucine | Leu |
| Arginine | Arg | Lysine | Lys |
| Asparagine | Asn | Methionine | Met |
| Aspartic acid | Asp | Phenylalanine | Phe |
| Glutamic acid | Glu | Proline | Pro |
| Glutamine | Gln | Serine | Ser |
| Glycine | Gly | Threonine | Thr |
| Histidine | His | Tryptophan | Trp |
| Isoleucine | Ile | Tyrosine | Tyr |
| Cysteine | Cys | Valine | Val |

The configuration of a particular amino acid, other than glycine, will be understood to be the natural L-configuration.

3

According to one feature of the present invention we thus provide a polypeptide of the formula I:-

$$X^1-X^2-X^3-X^4-X^5-X^6-X^7-X^8-X^9-X^{10}-X^{11}-X^{12}-X^{13}$$
$$X^{14}-X^{15}-X^{16}-Leu-Leu-Ala-Gln-X^{21}-X^{22}-X^{23}-Ile-Ser-X^{26}$$
$$X^{27}-X^{28}-X^{29}-X^{30}-X^{31}-X^{32}-Arg-X^{34}-Asp-Phe-X^{37}-X^{38}-Pro$$
$$Gln-Glu-Glu-Phe-X^{43a}-X^{44}-Asn-Gln-Phe-Gln-Lys-X^{50}-X^{51}$$
$$X^{52}-Ile-X^{54}-Val-Leu-His-Glu-X^{59}-Ile-X^{61}-Gln-X^{63}$$
$$X^{64}-Asn-Leu-X^{67}-X^{68}-Thr-X^{70}-Asp-Ser-Ser-Ala-Ala-Trp$$
$$X^{77}-X^{78}-X^{79}-Leu-Leu-X^{82}-Lys-Phe-X^{85}-Thr-Glu-Leu-Tyr$$
$$Gln-Gln-Leu-Asn-X^{94}-Leu-Glu-Ala-X^{98}-X^{99}-X^{100}-X^{101}$$
$$X^{102}-X^{103}-X^{104}-X^{105}-X^{106}-X^{107}-X^{108}-X^{109}-X^{110}-X^{111}$$
$$X^{112}-X^{113}-X^{114}-X^{115}-Ile-Leu-Ala-Val-X^{120}-Lys-Tyr-Phe$$
$$X^{124}-Arg-Ile-Thr-Leu-Tyr-Leu-X^{131}-Glu-Lys-Lys-Tyr$$
$$Ser-X^{137}-X^{138}-Ala-Trp-Glu-Val-Val-Arg-Ala-Glu$$
$$Ile-X^{148}-Arg-Ser-X^{151}-Ser-X^{153}-Ser-X^{155}-X^{156}-X^{157}$$
$$X^{158}-X^{159}-X^{160}-X^{161}-X^{162}-X^{163}-X^{164}-X^{165}.$$

in which $X^1$, $X^2$, $X^3$, $X^4$, $X^5$, $X^5$, $X^7$, $X^5$, $X^5$, $X^{10}$, $X^{11}$, $X^{155}$, $X^{156}$, $X^{157}$, $X^{158}$, $X^{159}$, $X^{160}$, $X^{161}$, $X^{162}$, $X^{163}$, $X^{164}$ and $X^{165}$ may each represent a naturally occurring alpha-amino acid and $X^{12}$ represents Arg or Ala, $X^{13}$ represents Arg or Ala, $X^{14}$ represents Thr or Ala, $X^{15}$ represents Leu or Ala, $X^{16}$ represents Met, Leu or Ile, $X^{21}$ represents Met or Leu, $X^{22}$ represents Ser, Arg or Gly and $X^{23}$ represents Arg or Lys, $X^{26}$ represents Leu or Pro, $X^{27}$ represents Ser or Phe, $X^{28}$ represents Ser or Ala, $X^{29}$ represents Ser or Cys, $X^{30}$ represents Leu or Ile $X^{31}$ represents Met or Lys, $X^{32}$ represents Ala, Asn, Asp or Glu, $X^{34}$ represents His or Pro, $X^{37}$ represents Gly, or Ala, $X^{38}$ represents Phe or Leu, $X^{43a}$ represents a single bond or Asp, $X^{44}$ represents Gly or Ala, $X^{50}$ represents Ala or Thr, $X^{51}$ represents Glu Pro or Glu Gln $X^{52}$ represents Ala or Thr, and $X^{54}$ represents Ser or Pro, $X^{59}$ represents Leu or Met, $X^{51}$ represents Gln or Ala, $X^{53}$ represents Ile or Thr, $X^{54}$ represents Phe or Ala, $X^{57}$ represents Phe or Ala, $X^{58}$ represents Thr or Ser, $X^{70}$ represents Lys or Glu, $X^{77}$ represents Asp or Glu, $X^{78}$ represents Glu or Gln, $X^{79}$ represents Asp, Thr or Ser, $X^{52}$ represents Asp or Glu $X^{55}$ represents Cys, Tyr or Ser, $X^{54}$ represents Asp or Asn, $X^{58}$ represents Cys or Leu, $X^{59}$ represents Val or Thr, $X^{100}$ represents Met, Ile or Asn, either $X^{101}$ represents Gln, Tyr or Phe, $X^{102}$ represents Glu, Gly, Ser or Ala, $X^{103}$ represents Glu, Val or Lys, $X^{104}$ represents Arg, Gly, Thr, Leu or Ala, $X^{105}$ represents Val, Asp, Met or Thr, $X^{106}$ represents Gly, Thr, Glu, Leu or Asn, $X^{107}$ represents Glu, Asn or Tyr, $X^{108}$ represents Thr or Val, $X^{109}$ represents Pro or Gln, $X^{110}$ represents Leu or Arg and $X^{111}$ represents Met, Lys or Leu,

or $-X^{101}-X^{102}-X^{103}-X^{104}-X^{105}-X^{106}-X^{107}-X^{108}-X^{109}-X^{110}-X^{111}-$ represents Met, Lys or Leu, $-X^{112}$ represents Asn, Lys or Ala, $X^{113}$ represents Ala, Glu or Ile, $X^{114}$ represents Asp or His, $X^{115}$ represents Ser or Phe, $X^{120}$

represents Lys or Arg, $X^{124}$ represents Arg or Gln, $X^{131}$ represents Thr or Lys, $X^{137}$ represents Pro or Ala, $X^{138}$ represents Cys or Ser, $X^{148}$ represents Met or Leu, $X^{151}$ represents Leu or Phe, $X^{153}$ represents Leu or Phe, the amino acids being selected such that an amino acid is present in at least one position selected from alpha$_2$ positions 12-16, 21, 28,29, 30, 32, 37, 44, 61, 64, 67, 98-115, 137, 138 and 148 which differs from the amino acid in the corresponding position of IFN-alpha$_2$, IFN-alpha$_1$ and IFN-alphaI and/or selected such that an amino acid is absent from at least one position selected from alpha$_2$ positions 101-110;

or the amino acids being selected such that an amino acid is present in at least one position selected from alpha$_2$ positions 27, 31, 59, 151, 160 and 163 which is the same as the amino acid in the corresponding position of IFN-alpha$_1$, the amino acids at alpha$_2$ positions other than positions 1-11, 27, 31, 43a, 59, 100, 102-104, 106, 112, 113, 151 and 156-165 being the same as the amino acids in the corresponding positions of IFN-alpha$_2$) or a $N_{1-11}$ and/or $C_{1-11}$ truncated analogue thereof and the $N_{3-11}$ truncated analogues of IFN-alpha$_2$, which may if desired be $C_{1-11}$ truncated, with the proviso that a $N_3$-truncated analogue of IFN-alpha$_2$ is also $C_{7-11}$ truncated.

It will be appreciated that the amino acids at positions 1 to 11 and 155 to 165 are selected so as not to destroy the overall conformation of the molecule. Thus it is preferred that a variety of different amino acids should precede the $N^{12}$ amino acid residue and follow the $N^{154}$ amino acid residue. Polypeptides in which all amino acid residues preceding $N^{12}$ and/or following $N^{154}$ are the same, especially where that amino acid is cysteine should be avoided. Similarly the presence of repeated (for example more than six) hydrophobic or repeated (for example more than six) charged amino acid residues in sequence in either the $N_{1-11}$ or $C_{1-11}$ portion of the polypeptide is preferably avoided.

In the polypeptide of formula I preferably $X^1$ represents Cys, $X^2$ represents Asp or Trp, $X^3$ represents Leu or Cys, $X^4$ represents Pro or Gln, $X^5$ represents Glu, Gln or Asp, $X^5$ represents Thr or Pro, $X^7$ represents His or Tyr, $X^5$ represents Ser, $X^5$ represents Leu, $X^{10}$ represents Asp, Gly or Ala, $X^{11}$ represents Asn, Ser or Ala, $X^{155}$ represents Thr, $X^{156}$ represents Asn, $X^{157}$ represents Leu, $X^{158}$ represents Gln, $X^{159}$ represents Glu or Lys, $X^{160}$ represents Arg, Ser or Ile, $X^{161}$ represents Leu, $X^{162}$ represents Arg, $X^{163}$ represents Arg or Ser, $X^{164}$ represents Lys and $X^{165}$ represents Glu or Asp.

It will be understood that references herein to an $N_{1-11}$ truncated analogue mean an analogue of the polypeptide of formula I which is shortened by the absence from the N-terminal end of the polypeptide of any of the first 1-11 amino acids without a sequence change. Preferably such references indicate an analogue of the polypeptide of formula I in which the first, first two or first three, amino acids are absent from the N-terminal end of the polypeptide of formula I. Similarly references herein to a $C_{1-11}$ truncated analogue mean an analogue of the polypeptide in which the C-terminal end of the polypeptide is shortened by the absence of any of the last 1-11 C-terminal amino acids without a sequence change being effected. Preferably such references indicate an analogue in which the last, last two, last three, last four, last five, last six, last seven, last eight, last nine or last ten amino acids are absent from the C-terminal end of the polypeptide.

It will be appreciated that the polypeptide of formula I may carry a methionine at its N-terminus. Moreover the polypeptide of formula I may carry additional amino acid residues at its N-terminus which in turn may be preceded by methionine. Preferably the polypeptide of formula I will carry no more than 17 additional amino acid residues at its N-terminus.

It will also be appreciated that the polypeptides of the present invention may if desired be radiolabelled. The radiolabel may for example be present at the sulphur of a methionine or it may be present as an iodine substituent on tyrosine.

The polypeptides of the present invention thus encompass certain $N_{3-11}$, truncated analogues of IFN-alpha$_2$ per se such as IFN-alpha$_2$ (10-165) and IFN-alpha$_2$ (12-165) as well as such analogues which are additionally $\overline{C_{1-11}}$ preferably $\overline{C_{1-10}}$ C-truncated such as IFN-alpha$_2$ (4-155). Indeed in our hands we have found that IFN-alpha$_2$ (4-155) has a particularly good relative antiviral potency in terms of the ratio of antiviral activity to antiproliferative activity and thus IFN-alpha$_2$ (4-155) is a preferred polypeptide of the invention.

In one particular embodiment, the present invention relates to polypeptides of formula I other than truncated analogues.

EP 0 194 006 B1

The present invention also encompasses for example polypeptides of the formula II:

```
Cys-Asp-Leu-Pro-X⁵ᵃ-Thr-His-Ser-Leu-X¹⁰ᵃ-X¹¹ᵃ-Arg-Arg-
-Thr-Leu-Met-Leu-Leu-Ala-Gln-Met-Arg-Lys-Ile-Ser-Leu-
-X²⁷ᵃ-Ser-Cys-Leu-X³¹ᵃ-Asp-Arg-His-Asp-Phe-Gly-Phe-Pro-
-Gln-Glu-Glu-Phe-X⁴³ᵃ-Gly-Asn-Gln-Phe-Gln-Lys-Ala-Glu-
-Tyr-Ile-Pro-Val-Leu-His-Glu-X⁵⁹ᵃ-Ile-Gln-Gln-Ile-Phe-
-Asn-Leu-Phe-Ser-Thr-Lys-Asp-Ser-Ser-Ala-Ala-Trp-Asp-
-Glu-Thr-Leu-Leu-Asp-Lys-Phe-Tyr-Thr-Glu-Leu-Tyr-Gln-

-Gln-Leu-Asn-Asp-Leu-Glu-Ala-Cys-Val-X¹⁰⁰ᵃ-Gln-
-X¹⁰²ᵃ-X¹⁰³ᵃ-X¹⁰⁴ᵃ-Val-X¹⁰⁶ᵃ-Glu- Thr-Pro-Leu-Met-
-X¹¹²ᵃ-X¹¹³ᵃ-Asp-Ser-Ile-Leu-Ala-Val-Arg-Lys-Tyr-Phe-
-Gln-Arg-Ile-Thr-Leu-Tyr-Leu-Lys-Glu-Lys-Lys-Tyr-Ser-
-Pro-Cys-Ala-Trp-Glu-Val-Val-Arg-Ala-Gln-Ile-Met-Arg-
-Ser-X¹⁵¹ᵃ-Ser-Leu-Ser-Thr-Asn-Leu-Gln-Glu-X¹⁶⁰ᵃ-
-Leu-Arg-X¹⁶³ᵃ-Lys-Glu
```

(in which X⁵ᵃ represents Glu or Gln, X¹⁰ represents Asp or Gly, X¹¹ represents Asn or Ser, X²⁷ᵃ represents Phe or Ser, X²¹ᵃ represents Lys or Met, X⁴³ᵃ represents Asp or a single bond, X⁵⁹ᵃ represents Met or Leu, X¹⁰⁰ᵃ represents Met or Ile, X¹⁰²ᵃ represents Glu or Gly, X¹⁰³ᵃ represents Glu or Val, X¹⁰⁴ᵃ represents Arg or Gly, X¹⁰⁶ᵃ represents Gly or Thr, X¹¹²ᵃ represents Asn or Lys, X¹¹³ᵃ represents Ala or Glu, X¹⁵¹ᵃ represents Leu or Phe, X¹⁶⁰ᵃ represents Arg or Ser and X¹⁶³ᵃ represents Arg or Ser with the proviso that the amino acids are selected such that at least one of X²⁷ᵃ, X²¹ᵃ, X⁵⁹ᵃ, X¹⁵¹ᵃ, X¹⁶⁰ᵃ and X¹⁶³ᵃ represents the same amino acid as that in the corresponding position of IFN-alpha₁) or a N₁₋₁₁ or C₁₋₁₁ truncated analogue thereof.

Preferred polypeptides of formula II by virtue of their improved relative antiviral potency in terms of the ratio of antiviral activity to antiproliferative activity are compounds of formula II in which X⁴³ᵃ represents Asp, X¹⁰⁰ᵃ represents Ile, X¹⁰²ᵃ represents Gly, X¹⁰³ᵃ represents Val, X¹⁰⁴ᵃ represents Gly, X¹⁰⁶ᵃ represents Thr, X¹¹²ᵃ represents Lys and X¹¹³ᵃ represents Glu with the proviso that at least one of X⁵ᵃ, X¹⁰ᵃ, X¹¹ᵃ, X¹⁵¹ᵃ, X¹⁶⁰ᵃ and X¹⁶³ᵃ represents an amino acid which differs from the amino acid in the corresponding position of IFN-alpha₂.

An especially preferred compound of formula II by virtue of its good ratio of antiviral to antiproliferative activity is endo-Asp⁴³ᵃ-[Leu¹⁵¹, Arg¹⁶⁰, ¹⁶³]IFN-alpha₂.

6

The polypeptides of the present invention relate in particular to analogues of IFN-alpha$_2$ of formula I in which an amino acid is present in at least one position selected from alpha$_2$ positions 12-16,21,28, 29,30,32,37,44,61,64,67,98-115,137, 138 and 148 which differs from the amino acid in the corresponding position of IFN-alpha$_2$, IFN-alpha$_1$ and IFN-alphaI.

In a further embodiment of the present invention there are provided compounds of formula I in which $X^1$ represents Cys, $X^2$ represents Asp or Trp, $X^3$ represents Leu or Cys, $X^4$ represents Pro or Gln, $X^5$ represents Gln or Asp, $X^5$ represents Thr or Pro, $X^7$ represents His or Tyr, $X^5$ represents Ser, $X^5$ represents Leu, $X^{10}$ represents Gly or Ala, $X^{11}$ represents Ser or Ala, $X^{16}$ represents Leu or Met, $X^{22}$ represents Arg, $X^{23}$ represents Lys, $X^{26}$ represents Leu, $X^{27}$ represents Phe, $X^{31}$ represents Lys, $X^{34}$ represents His, $X^{38}$ represents Phe, $X^{43a}$ represents a single bond, $X^{50}$ represents Ala, $X^{51}$ represents Glu, $X^{52}$ represents Thr, $X^{54}$ represents Pro, $X^{53}$ represents Ile, $X^{58}$ represents Ser, $X^{70}$ represents Lys, $X^{77}$ represents Asp, $X^{78}$ represents Glu, $X^{79}$ represents Thr, $X^{52}$ represents Asp, $X^{55}$ represents Tyr, $X^{54}$ represents Asp, $X^{105}$ represents Val, Asp or Thr, $X^{106}$ represents Thr, Gly, Leu or Asn, $X^{120}$ represents Arg, $X^{124}$ represents Gln, $X^{131}$ represents Lys, $X^{151}$ represents Phe, $X^{153}$ represents Leu, $X^{156}$ represents Asn, $X^{157}$ represents Leu, $X^{158}$ represents Gln, $X^{159}$ represents Glu, $X^{160}$ represents Ser, $X^{161}$ represents Leu, $X^{162}$ represents Arg $X^{163}$ represents represents Lys and $X^{165}$ represents Glu) and the $N_{1-11}$ and $C_{1-11}$ truncated analogues thereof. Where such truncated analogues are obtained they are advantageously the $N_{1-3}$ and/or $C_{1-10}$ truncated analogues.

Generally it is preferred that the compounds of formula I of the present invention contain a Cys$^{29}$, Cys$^{138}$ disulphide bridge as in natural IFN-alpha$_2$. We have found that [Ser$^{29}$,Ser$^{138}$]-IFN-alpha$_2$, however, not only possesses antiviral activity, but possesses a relative antiviral potency in terms of the ratio between antiviral and antiproliferative activity (AV/AP) which is substantially greater than that reported for Hu-IFN-alpha$_2$. This is a particularly surprising finding in view of the belief in the literature that the Cys$^{29}$, Cys$^{138}$ disulphide bridge is essential for activity.

7

Compounds of the formula:-

$$\text{Cys } X^2 X^3 X^4 X^5 X^6 X^7 \text{-Ser-Leu-} X^{10c} \text{-Ser-Arg-Arg-Thr-Leu-Met}$$

$$\text{Leu-Leu-Ala-Gln-Met-Arg-Lys-Ile-Ser-Leu-Phe-Ser-Cys}$$

$$\text{Leu-Lys-Asp-Arg-His-Asp-Phe-} X^{37} \text{-Phe-Pro-Gln-Glu-Glu}$$

$$\text{Phe-} X^{44} \text{-Asn-Gln-Phe-Gln-Lys-Ala-Glu-Thr}$$

$$\text{Ile-Pro-Val-Leu-His-Glu-} X^{59} \text{-Ile-Gln-Gln-Ile-Phe-Asn}$$

$$\text{Leu-Phe-Ser-Thr- Lys-Asp-Ser-Ser-Ala-Ala-Trp-Asp-Glu}$$

$$\text{Thr-Leu-Leu-Asp-Lys-Phe-Tyr-Thr-Glu-Leu-Tyr-Gln-Gln}$$

$$\text{Leu-Asn- Asp-Leu-Glu-Ala-} X^{98c} \text{-} X^{99c} \text{-} X^{100c} \text{-} X^{101c} \text{-}$$

$$\text{-} X^{102c} \text{-} X^{103c} \text{-} X^{104c} \text{-} X^{105c} \text{-} X^{106c} \text{-} X^{107c} \text{-} X^{108} \text{-} X^{109} \text{-} X^{110}$$

$$X^{111c} \text{-} X^{112c} \text{-} X^{113c} \text{-} X^{114} \text{-Ser-Ile-Leu-Ala-Val-Arg-Lys}$$

$$\text{Tyr-Phe -Gln-Arg-Ile-Thr-Leu-Tyr-Leu-Lys-Glu-Lys-Lys}$$

$$\text{Tyr-Ser-Pro-Cys-Ala-Trp-Glu-Val-Val-Arg-Ala-Glu-Ile}$$

$$\text{Met- Arg-Ser-Phe-Ser-Leu-Ser-Thr-Asn-Leu-Gln-Glu-Ser}$$

$$\text{Leu-Arg-Ser-Lys-Glu}$$

[(wherein $X^2$ represents Asp or Trp, $X^3$ represents Leu or Cys, $X^4$ represents Pro or Gln, $X^5$ represents Gln or Asp, $X^5$ represents Thr or Pro, $X^7$ represents His or Tyr, $X^{10c}$ represents Gly or Ala, $X^{37}$ represents Gly or Ala, $X^{44}$ represents Gly or Ala, $X^{59}$ represents Met or Leu, $X^{98c}$ represents Cys or Leu, $X^{99c}$ represents Val or Thr, $X^{100c}$ represents Ile, Met or Asn, $X^{101c}$ represents Gln or Tyr, $X^{102c}$ represents Gly, Ala or Ser, $X^{103c}$ represents Val, $X^{104c}$ represents Gly, Ala or Thr, $X^{105c}$ represents Val or Asp, $X^{106c}$ represents Thr or Leu, $X^{107c}$ represents Glu or Asn, $X^{108}$ represents Thr or Val, $X^{109}$ represents Pro or Gln, $X^{110}$ represents Leu or Arg, $X^{111c}$ represents Met or Lys, $X^{112c}$ represents Lys or Ala, $X^{113c}$ represents Glu or Ile and $X^{114}$ represents Asp or His) and corresponding polypeptides in which Cys at positions 29 and 138 is replaced by Ser, the amino acids being selected such that at least in one of positions 2-7,10,37,44,59 and 98-114 an amino acid is present which differs from the amino acid in the corresponding position of IFN-alpha$_2$] and the $N_{1-11}$ (preferably $N_{1-3}$) and $C_{1-11}$ (preferably $C_{1-10}$) truncated analogues thereof are preferred because of their good relative antiviral potency in terms of the ratio of antiviral to antiproliferative activity.

The following features either alone or in combination are preferred;

1) the presence of gamma 98-114, especially gamma 101-114, in the Hu-IFN-alpha$_2$ polypeptide at positions 98-114, especially 101-114 respectively;

2) the presence of alanine in at least one of positions 10,37,44,102 and 104 in the Hu-IFN-alpha$_2$ polypeptide;

3) the presence of serine at both positions 29 and position 138 of the Hu-IFN-alpha$_2$ polypeptide;

4) the presence of leucine at position 59 of the Hu-IFN-alpha$_2$ polypeptide.

8

When any one of these features is taken either singly or in combination, with the other general or particular features of the polypeptides of the present invention, preferred sub-groups of polypeptides are obtained.

Especially preferred polypeptides by virtue of their good relative antiviral potency include:

$$[\text{gamma } (98-114)^{98-114}]\text{IFN-alpha}_2,$$
$$[\text{Met}^{100}, \text{gamma}(101-114)^{101-114}]\text{IFN-alpha}_2,$$
$$[\text{gamma } (2-7), \text{ gamma } (98-114)]\text{IFN-alpha}_2,$$
$$[\text{Leu}^{59}]\text{IFN-alpha}_2,$$
$$[\text{Ala }^{10,37,44,102,104}]\text{IFN-alpha}_2 \text{ and}$$
$$[\text{Ser}^{29,138}]\text{IFN-alpha}_2.$$

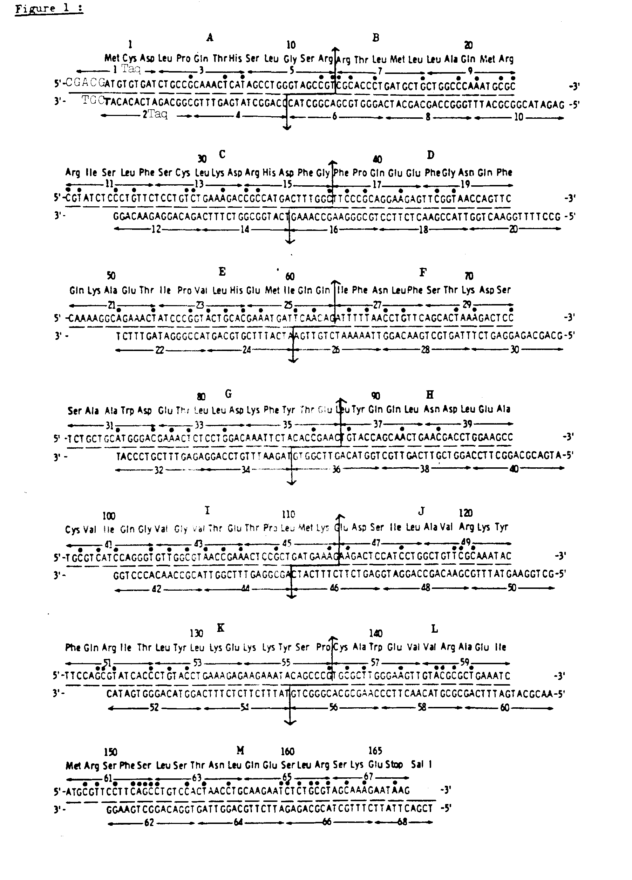

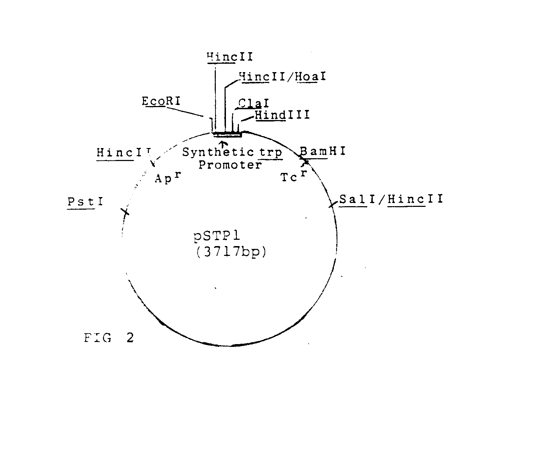

In the drawings accompanying this specification Figure 1 illustrates the nucleotide sequence of a Hu IFN-alpha$_2$ gene and the corresponding amino acid sequence; Figure 2 is a diagramatic representation of the structure of the plasmid pSTP1.

The polypeptides of the present invention are conveniently prepared by genetic engineering techniques for example such as described in European Patent Publication No. 0062971A2.

We thus provide according to a further feature of the present invention a process for producing a polypeptide of formula I as hereinbefore defined which process comprises culturing a microorganism, the microorganism having been transformed with a replicable plasmidic expression vehicle comprising genetic material coding for the said polypeptide of formula I, whereby to effect expression of the said polypeptide and recovering the said polypeptide thereby expressed.

The above-mentioned process may be effected by the use of any appropriate microorganism such as a gram negative E. coli or an autotrophic bacterium (e.g. one capable of using as its sole or principal carbon source inorganic carbon e.g. carbon dioxide, or a bicarbonate), or a methylotrophic bacterium (e.g. one capable of using as its sole or principal carbon source methane or 'methyl' carbon, for example from methanol). However we do not exclude the use of any of the microorganisms referred to in European Patent Publication No. 0062971A2.

Both anaerobic and aerobic varieties of microorganisms are found to be interest in the process of the present invention, as well as both facultative and obligate organisms, and the selection of an organism will be made in the light of the preferred processing conditions as well as the biochemical or biosynthetic capabilities of an organism having otherwise acceptable characteristics.

Preferred microorganisms for use in the above-mentioned process of the present invention include E. coli K-12 (JA 221) and especially E. coli K-12 (DS410). These microorganisms, JA 221 and DS410, are well known having the genotype F⁻ thiA leuB6 trpE 5 recA and F⁻ ara azi ton A lac Y min A min B rps L mal A xyl mtl thi respectively and are freely available to the public, having been freely available to the public since before 1 February 1985. Moreover E. coli JA 221 and E. coli DS 410 have been deposited by us, under the Budapest Treaty, with The National Collections of Industrial & Marine Bacteria Ltd, Torry Research Station, PO Box No. 31, 135 Abbey Road, Aberdeen, AB9 8DG Scotland under the deposition numbers NCIB 12099 and NCIB 12100 respectively. The deposition date in respect of each microorganism being 7 June 1985.

Typically, such micro-organisms may be aerobically cultured in an aqueous medium containing as a source of assimilable carbon, together with necessary inorganic nutrients. Generally, where, for example, the interferon analogue is not passed out of the cell at a commercially useful rate, the organism may be cultured and harvested as the intact cell conveniently with subsequent extraction of the cells for example after separation from the medium. Where the interteron analogue is passed out of the cell into the surrounding culture solution, however, the analogue may be extracted therefrom in the conventional way. Such techniques are well known and require no further explanation.

According to a further feature of the present invention there is thus provided a transformant microorganism capable of expressing a polypeptide of formula I as hereinbefore defined, the said microorganism comprising a replicable plasmidic expression vehicle, which vehicle comprises genetic material coding for the said polypeptide.

The transformant microorganism is preferably an E. coli JA221 transformant, especially an E. coli DS410 transformant.

According to a further feature of the present invention there is provided a process for the preparation of a tranformant microorganism as hereinbefore defined which comprises tranforming a microorganism by the insertion therein of a replicable plasmidic expression vehicle, which vehicle comprises genetic material coding for a polypeptide of formula I as hereinbefore defined.

Methods for the introduction of foreign genetic material into microorganisms have been widely described in the literature. Such methods comprise formation of a replicable plasmidic expression vehicle which vehicle comprises a vector and the foreign genetic material, and introduction of the vehicle into the microorganism. Introduction of the vehicle into the microorganism may be advantageously facilitated by subjecting the microorganism to an appropriate treatment, for example treatment with a calcium chloride solution. Where the genetically modified microorganism of the present invention comprises a methylotroph or an autotroph the replicable plasmidic expression vehicle comprising the gene of an interferon analogue of the present invention is preferably introduced thereto by transfer from another organism by cell conjugation, more preferably by transfer of the replicable plasmidic expression vehicle from a strain of E. coli.

According to a further feature of the present invention we provide a replicable plasmidic expression vehicle capable, in a transformant microorganism, of expressing a polypeptide of formula I as hereinbefore defined. We also provide according to a still further feature of the present invention a process for the preparation of such a replicable plasmidic expression vehicle which comprises inserting a gene coding for a polypeptide of formula I as hereinbefore defined or inserting a double stranded DNA fragment encoding a portion of the said polypeptide into a vector therefor at an appropriate insertion site whereby a replicable plasmidic expression vehicle is obtained capable of directing the synthesis of a polypeptide of formula I as hereinbefore defined in a transformant microorganism.

Thus in one embodiment of the present invention the replicable plasmidic expression vehicle may be prepared by inserting a gene coding for a polypeptide of formula I into a vector therefor. In a further embodiment a vector comprising a promoter sequence and a nucleotide sequence or nucleotide sequences which code for a portion of the polypeptide of formula I is used and the nucleotide sequence fragment(s) coding for the remainder of the polypeptide of formula I is (are) inserted into the vector to form the replicable plasmidic expression vehicle.

The replicable plasmidic expression vehicle of the present invention is advantageously prepared by the use of a plasmid vector comprising an E. coli lac or trp promoter, preferably a plasmid vector as described in European Patent Publication No. 0062971.

Expression of the gene encoding for the polypeptide of formula I is for example effected under the influence of a trp promoter (the promoter of the E. coli tryptophan operon).

A synthetic trp promoter based on the tryptophan operon of E. coli K12 is conveniently employed and is present in the plasmid vector pSTP1 (Windass et al., Nucleic Acids Research, Vol. 10 (1983) p 6639) illustrated in Fig 2. Such a plasmid vector is of particular interest since it allows the gene coding for the polypeptides of formula I to be introduced between the ClaI and SalI sites of the plasmid vector just downstream of a potentially strong promoter.

The trp promoter together with such a gene (the Eco RI - SalI segment) may be removed from the vector and used as a portable expression unit, which unit constitutes a further feature of the present invention.

According to a further feature of the present invention there is provided a DNA sequence that encodes for a polypeptide of formula I as hereinbefore defined. Oligonucleotides selected from the aforesaid DNA sequence also constitute a further feature of the present invention.

The aforesaid DNA sequence and oligonucleotides may be prepared by any convenient process known per se, but one method which we have found advantageous for preparing the aforesaid DNA sequence and which constitutes a still further feature of the present invention comprises ligating appropriate oligonucleotides whereby to form a DNA sequence of the present invention.

The degeneracy of the genetic code permits substantial freedom in the choice of codons which can be used to construct a gene for the appropriate polypeptide of the present invention. Codons were normally chosen as those preferred in the expression of microbial genomes in a particular host such as E. coli. This consideration led to the preferred nucleotide sequences of the present invention which were selected to enable the polypeptides of the present invention to be expressed, such as the nucleotide sequence detailed in any one of the Examples hereinafter.

We have found that the synthesis of the gene of the polypeptides of the present invention may be facilitated by synthesizing oligonucleotides, for example comprising about 14 or 15 nucleotides, and then ligating oligonucleotides, for example 4-6 oligonucleotides, in the appropriate sequence to form fragments, which fragments may then be assembled to form sequences and the sequences ligated to form the gene.

The DNA sequence that encodes for the polypeptides of formula I is for example flanked by appropriate initiation and termination codons and appropriate single stranded termini to allow ligation at a suitable site in a plasmid vector. The DNA sequence is thus preceded by for example:-

```
                        5' CGACGATG

                        3'    TGCTAC
```

which sequence comprises the initiation codon for methionine and the linker nucleotides which enable the DNA sequence to be inserted at a ClaI/TaqI restriction site. The DNA sequence may for example be followed by:-

```
                        5' TAAG

                        3' ATTCAGCT
```

which sequence comprises a termination codon and the linker nucleotides which enable linkage to be effected to the vector for example at the SalI site. In the preparation of the DNA sequences of the present invention these sequences are preferably included in the appropriate 5' and 3' oligonucleotides where it is desired to prepare the DNA sequence by the above-described technique of ligating oligonucleotides to form sequences which are then ligated to form fragments and the fragments ligated to form the DNA sequence or gene.

We do not however exclude the possibility that alternative synthetic strategies may be used. For example, the degeneracy of the genetic code permits restriction sites to be present in the nucleotide sequence or the gene and this may be used to facilitate gene synthesis.

According to a further feature of the present invention we therefore provide a vector comprising a promoter sequence and a nucleotide sequence or nucleotide sequences which code for a portion of the polypeptide of formula I, the vector being such that the nucleotide fragment coding for the remainder of the polypeptide of formula I may be inserted into the vector whereby to form replicable plasmidic expression vehicle of the present invention.

The nucleotide sequence of the vector at the site for insertion of the aforesaid gene fragment will thus be a restriction site(s) so that a restriction endonuclease may be used to cut the vector at the appropriate site for insertion therein of the said gene fragment. Thus, for example, the nucleotide sequence set out in Figure 1 has a Bst E II/Eca I restriction site (GGTAACC) at the codons for amino acids 44 to 46. Thus, where it is desired to effect changes to the IFN-alpha$_2$ polypeptide within the first 43 amino acids, only the section of the gene spanning the restriction site at the 5' end of the gene and the Bst EII/Eca I restriction site need be prepared. This section of the gene or fragment is referred to herein as a Cla-BstE fragment. The replicable plasmidic expression vehicle may thus be prepared directly by inserting the appropriate Cla-Bst E fragment into a vector containing an appropriate promoter sequence, e.g. a trp promoter and the IFN alpha$_2$ gene sequence between the Bst EII/Eca I site and the 3' end of the gene at the appropriate site. The Cla-Bst E fragments may, for example, be prepared by the production of a DNA sequence, which contains the desired restriction sites, the sequence then being cleaved by digestion with the appropriate restriction endonuclease but, may also be prepared using modified terminal fragments so that the single-stranded cohesive end for Bst EII/Eca I is already present.

The use of restriction site(s) in this way thus enables the genes for a series of polypeptides, modified in a particular region, to be produced in a replicable plasmidic expression vehicle without the need for the total synthesis of each gene. Moreover several vectors spanning any combination of restrictions site(s) may be generated and modified genes prepared by such procedures can be used to generate any combination of these modifications by transferring restriction fragments between plasmidic vectors. By way of example restriction sites introduced into the IFN-alpha$_2$ gene to facilitate modified gene synthesis are illustrated in the Table below:-

| Oligonucleotide Changed | New sequence (5'-3') | Restriction site introduced | New small sub-fragment |
|---|---|---|---|
| 4 | 4 SauI CTAAGGAATGAGTTG | SauI | A-SauI |
| 5 | 5 SauI TCCTTAGCTAAGGT | | |
| 29 | 29 XhoI AGCACTGAAGACTCG | XhoI | F-XhoI |
| 30 | 30 XhoI GCAGCACTCGAGTCT | | G-XhoI |
| 31 | 31 XhoI AGTGCCTGCATGGAC | | |
| 35 | 35 SstI AAATTCTACACGGAG | SstI | G-SstI |
| 36 | 36 SstI TCGTAGAGCTCGGTG | | H-SstI |
| 37 | 37 SstI CTCTACCAGCAAGTC | | G-Xho/SstI |
| 38 | 38 XbaI AGATCGTCAGTTGC | XbaI | H-XbaI |
| 39 | 39 XbaI AAGGATCTAGAAGCC | | H-Sst/XbaI |
| 40 | 40 XbaI ATGACGCAGCCTTCT | | |
| 60 | 60 BglII ATCTCATCAGTTTCAG | BglII | L-BglII |
| 61 | 61 BglII ATGAGATCTTTCAG | | M-BglII |
| 62 | 62 BglII TCGACAGGCTGAAAG | | |

The polypeptides of formula I as hereinbefore defined may be used as obtained or purified in a known or appropriate manner and formulated into pharmaceutical compositions for example by admixture with an appropriate inert or active diluent, which typically will be liquid, but may be solid.

The polypeptides of the present invention are potentially useful as antiviral, or anticancer agents or as modulators in the immune response of, for example, mammals and particularly man.

Various aspects of the present invention will now be described with reference to the following Examples which are illustrative of the invention.

**Examples 1-31**

**Preparation of HuIFN-alpha₂ analogues.**

**A. Preparation of the genes of HuIFN-alpha₂ analogues.**

IFN genes were prepared from oligodeoxyribonucleotides (oligonucleotides) according to the basic strategy outlined by Edge et al (Nature 1981, 292 756-762 and Nucleic Acid Res. 1983 11, 6419-6435) and in European Patent Publication No. 62971A2. These Examples describe the preparation of the genes of HuIFN-alpha₂ analogues which can be ligated to the plasmid vector pSTP 1, containing a synthetic trp promoter, to give expression of the gene in E. coli. 1.

**Oligonucleotide synthesis**

Oligonucleotides were prepared either by a solid-phase synthesis procedure as described in European Patent Publication No. 62971 A2 or by using the reagents and solvents described in the aforementioned patent application with the composite polydimethylacrylamide-Kieselguhr support in a continuous flow assembly apparatus as described by M. J. Gait et al. (J. Chem. Soc., Chem. Commun. 1982. p. 37-40). Crude oligonucleotides were purified by ion-exchange and reverse-phase high-performance liquid chromatography essentially as described by C. R. Newton et al. (Anal. Biochem. 1983 129, 22-30).

For the purposes of these Examples the structure of the oligonucleotides referred to herein as 1 Taq, 2 Taq and by a number of from 3 to 68 are as identified by the horizontal arrows appearing above and below the relevant oligonucleotide sequence in Fig 1 which depicts a synthetic interferon alpha₂ sequence. Where it is desired to refer to an oligonucleotide comprising an amino acid modification the same number will apply to the modified oligonucleotide as applied to the corresponding unmodified oligonucleotide with the proviso that additional notation is used to distinguish the modified oligonucleotide and its structure is identified hereinafter. It will further be noted that oligonucleotides 1 Taq and 2 Taq referred to herein differ from oligonucleotides 1 and 2 identified in Fig 2 of European Patent Publication No. 62971A2 and the designation 1 Taq and 2 Taq has been used herein to avoid confusion. Thus oligonucleotides 1 Taq and 2 Taq possess the following sequences:-

```
1 Taq 5' CGACGATGTGTGAT 3'
2 Taq  3' TGCTACACACTAGACGGC 5'
```

which sequences may be further modified if it is desired to code for amino acid(s) which differ from any of the first five amino acids of IFN-alpha₂ or if it is desired to omit any such amino acids.

2. **Ligation of chemically synthesised oligonucleotides**

Oligonucleotides were assembled into small DNA sub-fragments A-M essentially as detailed in Table 1 below:-

Table 1

| Fragments | Oligonucleotides |
|-----------|------------------|
| A | 1-5 |
| B | 6-10 |
| C | 11-15 |
| D | 16-20 |
| E | 21-25 |
| F | 26-30 |
| G | 31-35 |
| H | 36-40 |
| I | 41-45 |
| J | 46-50 |
| K | 51-55 |
| L | 56-60 |
| M | 61-68 |

The oligonucleotides were assembled into the small DNA sub-fragments A-M either as described by Edge et al. (Nucleic Acids Res. 1983 11, 6419-6435) and in European Patent Publication No. 62971A2 or by the following modification as illustrated in the preparation of small sub-fragment B:-

Oligonucleotides 6,7,8 and 9 (400 pmol. each) (Fig. 1) were separately phosphorylated with poly-nucleotide kinase and [gamma-$^{32}$P]ATP as described in the aforementioned European Patent Publication then mixed in the ratio 1:0.9:0.8:0.9 and ethanol precipitated. The precipitate was dissolved in water (50 $\mu$l) and the solution heated at 100°C. for 2 minutes to destroy residual kinase activity. Oligonucleotide 10 (400 p mol.) was added and the mixture extracted with butanol and then lyophilised. The residue was annealed and ligated to give fragment B as described in the aforementioned European Patent publication.

Small sub-fragment A was similarly prepared from oligonucleotides 1 Taq, 2 Taq, 3, 4, and 5 with oligonucleotide 1 Taq left unphosphorylated, to give a fragment subsequently referred to as A-taq.

3. **Assembly of A to M into the Interferon Gene**

Sequences (I to III M) were constructed by similarly ligating fragments A-taq to M after phosphorylating the 5'-hydroxy termini of fragments B to L, with T4 polynucleotide kinase and ATP (containing 0.15% of [gamma-$^{32}$P]ATP in groups as shown in Table 2.

Table 2

| Sequence | Fragments |
|----------|-----------|
| I | A-Taq, B, C, D |
| II | E, F, G, H |
| III | I, J, K, L, |
| IIIM | I, J, K, L, M |

The sequences I, II and III or IIIM were purified by gel electrophoresis in a 10% polyacrylamide slab and either I + II + III + M or I + II + IIIM ligated to give the gene of the appropriate Hu IFN-alpha$_2$ analogue. After precipitation the product mixture was phosphorylated with T4 polynucleotide kinase as described previously.

The following HuIFN-alpha$_2$ analogues were prepared by ligation of the oligonucleotides specified in the Table, according to the procedure described in this Example above and according to the basic strategy described in Edge et al (Nature 1981, 292, 756-762 and, Nucleic Acids Res. 1983 11, 6419-6435) and in European Patent Publication No. 62971A2. As stated above the structure of the oligonucleotides referred to below as 1 Taq, 2 Taq and by a number of from 3 to 68 are identified by the horizontal arrows above and below the oligonucleotide sequence in Fig 1. References herein to gamma interferon refer to the amino acid sequence detailed in "Structure of the human immune IFN gene" P W Gray or D V Goeddel Nature 298 859 (1982).

TABLE 3

| Ex No. | Name | New oligonucleotide | | New Small sub Fragment | oligonucleotides used. |
|---|---|---|---|---|---|
| | | Code | sequence (5'-3') | | |
| 1. | endo-Asp$^{43a}$-[Glu$^5$,Asp$^{10}$,Asn$^{11}$]IFN-$\alpha_2$ | 1 taq | CGACGATGTGTGAT | A-B1 | 1-taq, 2B1,x4,x5,x6 |
| | | 2-B1 | TCCGGCAGATCACACATCTG | B-B1 | x7,x8,8,9,10 |
| | | x4 | CTGCCGGAGACCCAT | | |
| | | x5 | CCAGGCTATGGGTC | | |
| | | x6 | AGCCTGGATAACCGT | | |
| | | x7 | AGGGTACGACGGTTAT | | |
| | | x8 | CGTACCCTGATGCTG | | |

EP 0 194 006 B1

| Ex No. | Name | New oligonucleotide | | New Small sub Fragment | oligonucleotides used |
|---|---|---|---|---|---|
| | | Code | sequence (5'-3') | | |
| 1. | | 18F8 | TTACCGTCGAACTCTTCC | D-F8 | 16,17,18F8,19F8,20 |
| | | 19F8 | TTCGACGGTAACCAGTTC | | |
| 2. | endo-Asp$^{43a}$-[Leu$^{151}$,Arg$^{160,163}$]IFN-$\alpha_2$ | F18 & F19 | see Example 1 | D-F8 | 16,17,18F8,19F8,20 |
| | | x62 | ATGCGTTCCCTGTCTT | M-B2 | x2,x62,x63,x64,x65,x66,x67 |
| | | x63 | GGTTGATAAAGACAGGG | | |
| | | x64 | TATCAACCAATCTTC | | |
| | | x65 | AAACGTTCTTGAAGATT | | |
| | | x66 | AAGAACGTTTAAGGCGC | | |
| | | x67 | TCGACTTATTCCTTGCGCCTT | | |
| | | x2 | AAGGAATAAG | | |
| 3. | [Y(2-7)$^{2-7}$]IFN-$\alpha_2$ | 1C5 | CGACGATGTGTTAC | | |
| | | 2C5 | CTGACAGTAACACATCGT | A-C5 | 1C5,2C5,3C5,4C5,5. |
| | | 3C5 | TGTCAGGACCCATAT | | |
| | | 4C5 | CCAGGCTATATGGGTC | | |
| 4. | [Y(98-114)$^{98-114}$]IFN-$\alpha_2$ | 40C4 | TTGGTCAGGGCTTCC | H-C4 | 36,37,38,39,40C4. |
| | | 41C4 | CTGACCAACTACTCT | I-C4 | 41C4,42C4,43C4,44C4,45C4 |
| | | 42C4 | TCAGTAACAGAGTAG | J-C4 | 46C4,47C4,48,49,50. |
| | | 43C4 | GTTACTGACCTGAAC | | |

| Ex No. | Name | New oligonucleotide | | New Small sub Fragments | oligonucleotides used. |
|---|---|---|---|---|---|
| | | Code | sequence (5'-3') | | |
| 4. | | 44C4 | CGCTGGACGTTCAGG | | |
| | | 45C4 | GTCCAGCGCAAAGCT | | |
| | | 46C4 | GAATGGATAGCTTTG | | |
| | | 47C4 | ATCCATTCCATCCTG | | |
| 5. | $[\text{Met}^{100}, \gamma(101\text{-}114)^{101\text{-}114}]\text{IFN-}\alpha_2$ | 41C6 | TGCGTCATGTACTCT | I-C6 | 41C6,42C6,43C4,44C4,45C4. |
| | | 42C6 | TCAGTAACAGAGTAC | J-C4 | Example 4 above. |
| 6. | $[\gamma(98\text{-}107)^{98\text{-}107}]\text{IFN-}\alpha_2$ | 44C7 | AGCGGAGTGTTCAGG | H-C4 | See Example 4 above. |
| | | | | I-C7 | 41C4,42C4,43C4,44C7,45. |
| 7. | $[\gamma(95\text{-}101)^{101\text{-}107}]\text{IFN-}\alpha_2$ | 41C8 | TGCGTCATCTTCGAG | | |
| | | 42C8 | GTCAGTTTCTCGAAG | | |
| | | 43C8 | AAACTGACCAACTAC | I-C8 | 41C8,42C8,43C8,44C8,45. |
| | | 44C8 | AGCGGAGTGTAGTTG | | |

EP 0 194 006 B1

| Ex No. | Name | New oligonucleotide | | New Small sub Fragments | oligonucleotides |
|---|---|---|---|---|---|
| | | Code | sequence (5'-3') | | |
| 8. | [Y(2-7),Met$^{100}$,Glu$^{102,103}$,Arg$^{104}$, Gly$^{106}$,Asn$^{112}$,Ala$^{113}$]IFN-$\alpha_2$ | 1-4C5 | see Example 3 for nucleotide sequence | A-C5 I-C3 | 1C5,2C5,3C5,4C5,5. 41C1,42C1,43C1,44C1,45C2. |
| | | 41 C1 | TGCGTCATGCAAGAG | | |
| | | 42 C1 | ACACGTTCCTCTTGC | | |
| | | 43 C1 | GAACGTGTAGGTGAG | | |
| | | 45 C2 | ACTCCGCTGATGAACG | | |
| | | 46 C2 | GAGTCTGCGTTCATC | J-C2 | 46C2, 47C2, 48, 49, 50 |
| | | 47 C2 | CAGACTCCATCCTG | | |
| 9. | [Y(2-7)Y(98-114)]IFN-$\alpha_2$ | 40-47C4 & 1-4C5 | see Examples 4 and 3 respectively for nucleotide sequences | A-C5 H-C4 I-C4 J-C4 | see Example 3 or 8. ) ) see Example 4 ) |
| | | 1C5 | | | |
| | | 2C5 | | | |
| | | 3C5 | | | |
| 10. | [Met$^{100}$,Y(2-7), Y(100-114)]IFN-$\alpha_2$ | 4C5 41C6 42C6 43-47C4 | see Examples 3, 4 & 5 for nucleotide sequences | A-C5 I-C6 J-C4 | see Examples 3 or 8. see Example 5. see Example 4. |
| 11. | [Leu$^{16,21,59,111,148}$]IFN-$\alpha_2$ | 7I2 8I2 | CGCACCCTGCTCCTG TGGGCCAGCAGGAGC | B-I2/3 | 6,7I2,8I2,9I3,10I3 |

| Ex No. | Name | New oligonucleotide | | New Small sub Fragment | oligonucleotides used. |
|---|---|---|---|---|---|
| | | Code | sequence (5'-3') | | |
| 11. | | 9I3 | CTGGCCCAACTGCGC | | |
| | | 10I3 | GAGATACGGCGCAGT | | |
| | | 24I4 | ATCAGTTCGTGCAGT | E-I4 | 21,22,23,24I4,25I4. |
| | | 25I4 | GAACTGATTCAACAG | | |
| | | 45I5 | ACTCCGCTGCTGAAAG | I-I5 | 41,42,43,44,45I5. |
| | | 46I5 | GAGTCTTCTTTCAGC | J-I5 | 46I5,47,48,49,50. |
| | | 60I6 | AACGCAGGATTTCAG | L-I6 | 56,57,58,59,60I6. |
| | | 61I6 | CTGCGTTCCTTCAGC | M-I6 | 61I6,62,63,64,65,66,67,68. |
| 12. | [Leu16]IFN-α2 | 7I2 | see Example 11 for nucleotide sequences. | B-I2 | 6,7I2,8I2,9,10 |
| 13. | [Leu21]IFN-α2 | 9I3 10I3 | see Example 11 for nucleotide sequences | B-I3 | 6,7,8,9I3,10I3. |
| 14. | [Leu59]IFN-α2 | 24I4 25I4 | see Example 11 for nucleotide sequences | E-I4 | see Example 11. |

EP 0 194 006 B1

| Ex No. | Name | New oligonucleotide Code | sequence (5'-3') | New Small sub Fragment | oligonucleotides used |
|---|---|---|---|---|---|
| 15. | [Leu[111]]IFN-$\alpha_2$ | 45I5 | see Example 11 for | I-I5 | see Example 11. |
|  |  | 46I5 | nucleotide sequences | J-I5 |  |
| 16. | [Ala[10,37,44,102,104]]IFN-$\alpha_2$ | 5D2 | AGCCTGGCTAGCCGT | A-D2 | 1 Taq,2 Taq,3,4,5D2 |
|  |  | 6D2 | AGGGTGCGACGGCTAG | B-D2 | 6D2,7,8,9,10. |
|  |  | 15D3 | CGCCATGACTTTGCT | C-D3 | 11,12,13,14,15D3. |
|  |  | 16D3 | TGCGGGAAAGCAAAG | D-D3/4 | 16D3,17,18D4,19D4,20. |
|  |  | 18D4 | TTAGCGAACTCTTCC | I-D5/6 | 41D5,42D5-D6,43D6,44,45. |
|  |  | 19D4 | TTCGCTAACCAGTTC |  |  |
|  |  | 41D5 | TGCGTCATCCAGGCT |  |  |
|  |  | 42D5-D6 | ACAGCAACAGCCTGG |  |  |
|  |  | 43D6 | GTTGCTGTAACCGAA |  |  |
| 17. | [Ala[137]]IFN-$\alpha_2$ | 55K10 | AAGAAATACAGCGCT | K-K10 | 51,52,53,54,55K10. |
|  |  | 56K10 | CAAGCGCAAGCGCTG | L-K10 | 56K10,57,58,59,60. |
| 18. | [Ser[29,138]]IFN-$\alpha_2$ | 12K1 | AGAGAGGAGAACAGG | C-K1 | 11,12K1,13K1,14,15. |

| Ex No. | Name | New oligonucleotide | | New small sub Fragment | oligonucleotides used. |
|---|---|---|---|---|---|
| | | Code | sequence (5'-3') | | |
| 18. | | 13K1 | TCCTCTCTGAAAGAC | | |
| | | 56K1 | CAAGCAGACGGGCTG | L-K1 | 56K1,57K1,58,59,60. |
| | | 57K1 | TCTGCTTGGGAAGTT | | |
| 19. | [Glu$^{32}$] IFN-$\alpha_2$ | 13K16 | TCCTGTCTGAAAGAG | C-K16 | 11,12,13K16,14K16,15. |
| | | 14K16 | TCATGGCGCTCTTTC | | |

21

| Ex No. | Name | New oligonucleotide | | New Small sub Fragment | oligonucleotides used |
|---|---|---|---|---|---|
| | | Code | sequence (5'-3') | | |
| 20. | [Ala$^{61,64,67}$] IFN-$\alpha_2$ | 26G3 | TTAGCAATCTGTGCA | | |
| | | 2763 | ATTGCTAACCTGGCT | F-G8 | 26G3, 27G3, 28G3, 29, 31 |
| | | 28G3 | TTAGTGCTAGCCAGG | | |
| 21. | IFN-$\alpha_2$(12-165) | 5A8 | CGACGATGCGT | B-A8 | 5A8,6A8,7,8,9,10[1] |
| | | 6A8 | AGGGTGCGACGCATCGT | | |
| 22. | IFN-$\alpha_2$(10-165) | 4A7 | ACCCATCGT | | |
| | | 5A7 | CGACGATGGGTAGCCGT | B-A7 | 4A7,5A7,6A7,8,9,10[1] |
| | | 6A7 | AGGGTGCGACGGCT | | |

EP 0 194 006 B1

| Ex No | Name | New oligonucleotide | | New Small sub Fragment | oligonucleotides used |
|---|---|---|---|---|---|
| | | Code | sequence (5'-3') | | |
| 23. | $Met^{100}$des(101-110)IFN-$\alpha_2$ | I | TGCGTCATGATGAAAG | | oligonucleotide I used in place of small sub frag- ment I N.B also converts Ile 100 to Met 100 |
| 24. | [Ala$^{10-15}$] IFN-$\alpha_2$ | 5All | AGCCTGGCTGCAGCT | A-All | 1-Taq,2-Taq,3,4,5All. |
| | | 6All | GCAGCAGCAGCTGCAG | B-All | 6All,7All,8,9,10 |
| | | 7All | GCTGCTGCGATGCTG | | |
| 25. | [Phe$^{115}$] IFN-$\alpha_2$ | 46J2 | AAGTCTTCTTTCATC | | |
| | | 47J2 | AAGACTTCATCCTG | J-J2 | 46J2,47J2,48,49,50 |

| Ex No. | Name | New oligonucleotide | | New Small sub Fragment | oligonucleotides used. |
| --- | --- | --- | --- | --- | --- |
| | | Code | sequence (5'-3') | | |
| 26. | IFN-)$_2$(4-155) | 2A1 | CGGCATCGT | A-A1 | 2A1,3A1,4,5 |
| | | 3A1 | CGACGATGCCGCAAACTCAT | | |
| | | 63A5 | CTGTCCACCTAAG | | |
| | | 64A5 | TCGACTTAGG | M-A5 | 61,62,63A5,64A5 |

EP 0 194 006 B1

| Ex No. | Name | Code | sequence (5'-3') | New Small sub Fragment | oligonucleotides used. |
|---|---|---|---|---|---|
| 27. | [Ala$^{32}$]IFN-$_2$ | 13K12 | TCCTGTCTGAAAGCT | C-K12 | 11,12,13K12,14K12,15 |
|  |  | 14K12 | TCATGGCGAGCTTTC |  |  |
| 28 | [Ile$^{30}$]IFN-$_2$ | 12K14 | ATACAGGAGAACAGG |  |  |
|  |  | 13K14 | TCCTGTATCAAAGAC | C-K14 | 11,12K14,13K14,14K14,15 |
|  |  | 14K14 | TCATGGCGGTCTTTG |  |  |
| 29. | [Asn$^{32}$]IFN-$_2$ | 13K17 | TCCTGTCTGAAAAAC | C-K17 | 11,12,13K17,14K17,15 |
|  |  | 14K17 | TCATGGCGGTTTTTC |  |  |
| 30 | [Ala$^{28}$]IFN-$_2$ | 12K9 | AGACAAGCGAACAGG | C-K9 | 11,12K9,13K9,14,15 |
|  |  | 13K9 | GCTTGTCTGAAAGAC |  |  |
| 31 | [Glu$^5$,Ser$^{27}$,Met$^{31}$,Leu$^{59}$]IFN | 3C14 | CTGCCGGAAACTCAT ) |  | 1 Taq,2 Taq, 3-C14, |
|  |  | 4C14SauI | CTAAGGAATGAGTTTC) | A-C14 Sau I | 4-C15 Sau I, 5 Sau I |
|  |  | 5 Sau I | TCCTTAGGTAGCCGT ) |  |  |
|  |  | 11C15 | CGTATCTCCCTGTCT) |  |  |
|  |  | 12C15 | AGACAGGAAGACAGG) |  |  |
|  |  | 13C16 | TCCTGTCTGATGGAC) | C-C15/16 | 11-C15, 12-C15, 14-C16, |
|  |  | 14C16 | TCATGGCGGTCCATC) |  | 15 |
|  |  | 24I4 | See Example 11 ) | E-I4 | See Example 14 |
|  |  | 25I4 | See Example 11 ) |  |  |

1. In respect of this Example there is no small sub fragment A for the gene.

## B. **Cloning of the synthetic genes of HuIFN-alpha$_2$ analogues**.

The synthetic genes of the interferon alpha$_2$ analogues listed in Table 3 were cloned into the plasmid vector, pSTP1 (Windass et al., Nucleic Acids Research, vol. 10, (1983) p6639), which included a promoter based on that of the tryptophan (trp) operon of E. coli K12.

For vector preparation, 200 $\mu$g. pSTP1 was dissolved in 168 $\mu$l. water, 2 $\mu$l. of 10 mg./ml. BSA and 20 $\mu$l. of 10 x restriction buffer (the restriction buffer is 6mM Tris. HCl pH 7.4, 6mM beta-mercaptoethanol 6mM MgCl$_2$, 10 $\mu$l. of the restriction endonuclease, Cla I (New England Biolabs, 5 units/$\mu$l.) was added and the mixture was incubated at 37°C. for 3 hours or more until linearised plasmid was predominant over supercoiled and nicked circular forms.

Linearised plasmid was separated from undigested plasmid molecules on a 1.4% preparative agarose gel. The band of linear plasmid was electroeluted into 5 ml. of 1/10 concentrated tris-borate electrophoresis buffer (from a 1 to 100 dilution of 108 g Tris base, 55 g. boric acid and 20 ml. 0.5M EDTA (pH8) made up to 1 litre with water). The eluate volume was reduced to about 50 $\mu$l. by repeated butanol extractions and diluted to 500 $\mu$l. with water. The eluate was extracted twice with 500 $\mu$l. of phenol: chloroform (1:1), twice with 1 ml. of water saturated ether and then concentrated to 20 $\mu$l. by butanol extractions. To this was added sequentially 160 $\mu$l. water, 20 $\mu$l. 3M sodium acetate (pH5.2) and 500 $\mu$l. of ethanol. The linear plasmid was allowed to precipitate overnight at -20°C. and pelleted by centrifugation in an Eppendorf microfuge for 15 minutes. The pellet was taken up in 140 $\mu$l. water and 20 $\mu$l. of 10 x restriction buffer was added followed by 20 $\mu$l. of 1 mg./ml. BSA and 15 $\mu$l. of 2M sodium chloride. 5 $\mu$l. of the endonuclease SalI (Boehringer, 10 units/ml.) was added and the mixture was incubated at 37°C. for at least three hours until the large ClaI-SalI fragment of pSTP1 was predominant. This fragment was prepared as above from a 1.4% preparative agarose gel. The ClaI-SalI vector fragment was then taken up in 1 ml. water and the DNA concentration was measured and adjusted to 47 $\mu$g./ml. For ligation, 14.9 $\mu$l. of vector DNA (0.7 $\mu$g.) was added to the synthetic interferon gene fragment (in water) and the DNA mixture was dried in vacuo. The DNA was taken up in 26.5 $\mu$l. of a mixture containing 525 $\mu$l. 2 x ligase buffer (132 mM Tris HCl. (pH 7.6)-,13.2 mM MgCl$_2$; 10 mM DTT and 0.4 mM ATP), 79 $\mu$l. ligase diluent (50 mM Tris HCl, pH 7.6, 10mM 2-mercaptoethanol; 500 $\mu$g./ml.BSA) 260 $\mu$l. 10 mM ATP, 33.5 $\mu$l. water and 30 $\mu$l. T4 DNA ligase (BRL, 2.5 Weiss units/$\mu$l.). Ligation was for 2 hours at 5°C. and then 12°C. overnight.

10 $\mu$l. of the DNA mixes were used directly to transform the strain JA221 using a modification of the standard procedure described by Mandel and Higa, Journal of Mol. Biol., 53 (1975) p154. The modifications involved the use of 0.1MCaCl$_2$ in 5mM Tris. HCl pH8 as the transformation buffer, a 45 minute incubation of the cells on ice following addition of DNA, a 30 minute incubation on ice after heat shock treatment and subsequent addition of L broth to 3.8 ml. Incubations prior to plating were overnight at room temperature without shaking. Aliquots of suspensions were plated onto L plates with 50 $\mu$g./ml. ampicillin. Transformants were screened for the presence of cloned synthetic genes of interferon analogues as follows:-

1. Colonies were streaked onto L plates with 10 $\mu$g./ml. tetracycline in order to identify transformants including the intact vector plasmid, pSTP1 which invariably did not include a gene for an interferon analogue.

2. Tetracycline sensitive transformants were analysed by colony hybridisation analysis using standard methods described in Molecular Cloning; A Laboratory Manual by Maniatis et al (Cold Spring Harbor).

For screening for recombinants including, for example, the synthetic interferon gene of Example 11, two separate hybridisations were performed using different small subfragments, one with a base sequence included in the natural interferon alpha$_2$ gene (group F), and the other with a sequence related to the synthetic interferon gene, for example to the synthetic interferon gene of Example 11 (subfragment BI2/3). 2 p moles of these subfragments were dried down and dissolved in 50 $\mu$l. of a mixture composed of 80 ul. of nick translation buffer (50 mM potassium phosphate pH 7.4, 5 mM MgCl$_2$), 32 $\mu$l. 200 $\mu$M. dATP, 32 $\mu$l. 200 $\mu$M dGTP, 32 $\mu$l. 200 $\mu$M dTTP, 25 $\mu$l. alpha$^{32}$P dCTP (10mCi./ml., 3,000Ci./mmol., Amersham), 579 $\mu$l. water and 20 $\mu$l. DNA polymerase I (12 units/$\mu$l. New England Biolabs). After 2 hours at room temperature, the labelled subfragments were isolated by Sephadex G$^5$0F chromatography using 10 mM Tris HCl. pH 8, 1 mM EDTA as running buffer. Lysis of colonies on filters and baking was performed as described in Molecular Cloning: A Laboratory Manual by Maniatis et al. (Cold Spring Harbor). Filters were prehybridised in 20 ul. Denhardts solution (a 50 fold dilution of Ficoll, polyvinyl pyrrolidine and BSA, each at 10 g./l. in water) at 65°C. for 1 hour. The $^{32}$P labelled subfragment probes were diluted into 2 volumes of 80% (v/v) formamide, 10 mM NaOH, 1mM EDTA, 0.05% (w/v)xylene cyanol, and 0.05% (w/v) bromophenol blue and heated for 2 minutes at 100°C. before cooling on ice.

The probe was added directly to the prehybridisation solution on the filters, left to hybridise overnight at 65°C. and air dried before autoradiography. The recombinants, for example, of the interferon gene of Example 11 were identified by positive hybridisation to both group F and group BI 2/3 subfragments.

3. The correct organisation of the interferon analogue gene was checked by restriction analysis using endonuclease HpaII. Thus, for example, analysis on a 6% polyacryamide gel showed that the interferon gene gave a pattern of HpaII fragments similar to that of the interferon alpha$_2$ gene.

3 interferon recombinants giving positive hybridisation signals were grown up on a small scale in order to select a final clone expressing the interferon analogue of interest for subsequent use in production of the analogue. These small scale grows were conducted as follows:-

Recombinants were grown overnight (250 rpm in a New Brunswick G24 shaking incubator at 37°C.) in 10 ml. Spizizens medium (1/10 dilution of $(NH_4)_2SO_4$ 20 g./l., $K_2HPO_4$ 106 g./l., $Na_3$ citrate 10 g./l. $KH_2PO_4$ 60 g./l., $MgSO_4.7H_2O$ 2g./l. pH 7.0 combined with 2/100 volume of a solution containing casein hydrolysate 25 g./l., L-leucine 1 g./l., thiamine 0.2 g./l., ampicillin 1.65 g./l. and glucose 100 g./l.) containing a high tryptophan concentration (100 mg./l.) and 50 ug./ml. ampicillin. Cells were pelleted and resuspended in 75 ml. Spizizens medium supplemented with a low tryptophan concentration of 2 mg./l. Incubation was for 5.5 hours at 37°C. at 250 rpm. Cells were pelleted at 4°C. and taken up in 2 ml. lysis buffer (15% sucrose, 50 mM. Tris. HCl. pH8 and 50 mM EDTA) and frozen overnight at -20°C. The cells were then thawed and 40 $\mu$l. lysosyme (50 mg. per ml. of lysis buffer) was added followed by 20 $\mu$l. 2.5% SDS. After incubation for 30 minutes on ice, 200 $\mu$l. of DNAase solution (66 $\mu$g./ml. DNAase in 0.15 M Tris. HCl. pH 7.5, 0.28 M $MgCl_2$, 40 mM $CaCl_2$) was, added and the lysate was incubated for 30 minutes on ice. The lysate was then sonisated for 90 seconds using the cup tip of a Heat Systems W-375 Sonicator set at full power. The lysate solution was clarified by centrifugation in a Sorvall SS34 rotor at 15,000 rpm for 20 minutes at 4°C. The supernatant was decanted and aliquoted for biochemical and biological evaluation of the interferon analogue.

## C. 1. Example of Production of Interferon Analogue Proteins in E. coli JA221.

A recombinant strain of E. coli JA221 expressing an IFN $\alpha_2$-related protein was recovered from stock, streaked on an L Agar plate supplemented with ampicillin (50 $\mu$g./ml.) and incubated at 37°C. for 17 hours. A loop of cells was tranferred from this plate into each of 4 x 250 ml. Erlenmeyer flasks containing 75 ml. Spizizen Medium 1, and these flasks shaken at 300 rpm on an orbital incubator at 37°C., overnight. Bacterial cells were harvested by centrifugation in 6 x 37.5 ml. aliquots in the SS34 rotor of a sorval RC5B centrifuge at 10,000 rpm for 6 minutes at room temperature, and resuspended in 3 ml. of prewarmed Spizizen Medium 2. These resuspended cells were then added to 6 x 750 ml. aliquots of Spizizen Medium 2 in 2L Erlenmeyer flasks and shaken at 250 rpm on an orbital incubator at 37°C. for 5.5 hours. At the end of this time, bacterial cells were harvested by centrifugation in the H6000A rotor of a Sorval RC3B centrifuge at 5,000 rpm for 20 minutes at 4°C, and resuspended in a total volume of 40 ml. lysis buffer (50mM EDTA, 15% (w/v) sucrose in 50 mM Tris-HCl pH 8.0). The resultant 40 ml cell suspension was stored at -20°C. prior to lysis.

### Media

Spizizen Medium 1 contained per litre of distilled water:$(NH_4)_2SO_4$: 2.0 g; $KH_2PO_4$: 10.6 g; $Na_3$ citrate: 1.0 g; $KH_2PO_4$; 6.0 g; $MgSO_4.7H_2O$: 0.2 g; casein hydrolysate: 0.5 g; L-leucine: 20 mg; thiamine: 4 mg; L-tryptophan: 100 mg; ampicillin: 33 mg; glucose: 2 g.

Spizizen Medium 2 was identical except that it contained L-tryptophan at 2 mg./l. and did not contain ampicillin.

L Medium contained per litre of distilled water: Bacto tryptone: 10 g; Bacto Yeast Extract: 5 g; NaCl: 5 g; Glucose 1 g; and when necessary, Bacto Agar: 15 g.

### 2. Production of Interferon Analogue Proteins in E. coli DS410

A plasmid preparation from the recombinant strain of E. coli JA221 referred to in B above is used to transform E. coli DS410 and the transformant, which expresses an IFN alpha$_2$ protein analogue, was streaked on an L Agar plate supplemented with ampicillin (50 $\mu$g./ml.) and incubated at 37°C. for 17 h. A loop of cells were transferred from this plate into each of 2 x 250 ml. Erlenmeyer flasks containing 75 ml. of L-broth supplemented with ampicillin (50 $\mu$g./ml.), and these flasks shaken at 300 rpm on an orbital incubator at 37°C, overnight. Bacterial cells were harvested by centrifugation in 4 x 37.5 ml. aliquots in the SS34 rotor of a Sorval RC5B centrifuge at 10,000 rpm for 6 minutes at room temperature, and resuspended in 3 ml. prewarmed Spizizen Medium 2. These resuspended cells were pooled in 63 ml. prewarmed Spizizen Medium 2, and 3.75 ml of the resultant suspension inoculated into each of 12 x 75 ml aliquots of Spizizen Medium 2 in 250ml. Erlenmeyer flasks which were subsequently shaken at 300 rpm on an orbital incubator at 37°C. for 7h. At the end of this time bacterial cells were harvested and processed as described in C1.

D. **Lysis of Bacteria and Extraction of Interferon Analogue Proteins.**

40 ml. of a frozen cell suspension was thawed to 4°C. and 800 $\mu$l. of a solution of lysozyme in lysis buffer (50 mg./ml.) added, immediately followed by 400 $\mu$l. LDS in distilled water (2.5% w/v). After incubation on ice for 30 minutes, 4 ml. DNAse solution (ca. 5 $\mu$g. DNAse dissolved in 150 mM Tris. HCl. pH 7.5 containing 280 mM $MgCl_2$ and 4 mM $CaCl_2$) was added and incubation on ice continued for a further 30 minutes. The resultant viscous material was subject to ultrasonic disintegration by 3 x 30s pulses from a 150 W MSE ultrasonic disintegrator set to deliver full power, and then centrifuged at 15,000 rpm for 15 minutes at 4°C. in the SS34 rotor of Sorval RC5B centrifuge. The supernatant from this centrifugation was then further centrifuged at 40,000 rpm for 1h. at 4°C. in the 50.2 Ti rotor of a Beckman L5-50B ultracentrifuge. The supernatant from this centrifugation contained the interferon analogue protein and was further purified as required.

E. **Purification**

1. **Purification by precipitation and size exclusion chromatography**

All operations were at 4°C. unless indicated otherwise.

Clarified supernatant (40 ml.) containing the interferon analogue was prepared as hereinbefore described.

Trichloroacetic acid (50% v/v, 2 ml.) was added dropwise with stirring to give a final concentration of 2.5%. (Alternatively saturated ammonium sulphate solution (80 ml.) was added dropwise with stirring over 15 minutes to give a final concentration of 50%). After stirring for 30 min and standing for 30 min, the precipitated protein was collected by centrifugation at 10,000 rpm for 30 min in an MSE High Speed 18 centrifuge.

The supernatant was discarded and the pellet extracted into 0.1M $K_2HP0_4$, pH8 (20 ml.) using a Ystral homogeniser. The milky suspension was adjusted to pH8 by dropwise addition of 2M NaOH with stirring using a Radiometer pH meter 26. The extract was ultracentrifuged at 34,000 rpm for 30 min in a 50.2.Ti rotor in a beckman L5-50B ultracentrifuge and the supernatant (21 ml.) applied immediately to a column (5 x 90 cm.) of Ultrogel AcA54 equilibrated in 0.1M $K_2HP0_4$, pH8 at 7°C. The column was eluted with the same buffer at a flow rate of 40 ml./hr. and fractions of approximately 12 ml. were collected. Fractions 95-125, known to contain the interferon analogue and excluding over 80% of total bacterial protein, were pooled (400 ml.) and concentrated to about 20 ml. in an Amicon Diaflo cell using an Amicon YM10 membrane. The retentate was filtered through a Millex GV, 0.22 um. filter unit and the sterile filtrate (19 ml.) was divided into multiple aliquots and stored at -20°C; a new aliquot was used for each assay.

Purification was monitored by antiviral and NK2-IRMA assays and the antiviral and NK2-IRMA titres for each relevant analogue are set out in Table 4 below. A hyphen in the NK2-IRMA assay column indicates that the relevant analogue was inactive in the NK2-IRMA assay.

**TABLE 4**

E. Gel filtration purified analogue profiles

| Example No. | Protein | Profile Sample Titre (U/Ml.) | |
|---|---|---|---|
| | | AV (x10$^6$) | NK-2IRMA (x10$^6$) |
| 3. | [γ(2-7)$^{2-7}$]IFN-γ$_2$ | 0.26 | 3.9 |
| 4. | [γ(98-114)98-114]IFN-γ$_2$ | 0.42 | — |
| 5. | [Met$^{100}$,γ(101-114)101-114]IFN-γ$_2$ | 0.09 | — |
| 6. | [γ(98-107)98-107]IFN-γ$_2$ | 0.22 | 5.3 |
| 8. | [γ(2-7),Met$^{100}$,Glu$^{102,103}$,Arg$^{104}$,Gly$^{106}$Asn$^{112}$Ala$^{113}$]IFN-γ$_2$ | 0.09 | — |
| 9. | [γ(2-7),γ(98-114)]IFN-γ$_2$ | 0.21 | — |
| 10. | [Met$^{100}$,γ(2-7),γ(101-114)]IFN-γ$_2$ | 0.05 | — |
| 11. | [Leu$^{16,21,59,111,148}$]IFN-γ$_2$ | 0.02 | 0.36 |
| 12. | [Leu$^{16}$]IFN-γ$_2$ | 0.54 | 3.9 |
| 13. | [Leu$^{21}$]IFN-γ$_2$ | 0.28 | 9.5 |
| 14. | [Leu$^{59}$]IFN-γ$_2$ | 0.02 | 0.35 |
| 15. | [Leu$^{111}$]IFN-γ$_2$ | 0.59 | 1.4 |
| 22. | IFN-γ$_2$(4-155) | 0.39 | 0.5 |

The interferon analogue protein still represents only about 0.1% of the total protein in the bioprofiling sample and as such cannot be distinguished directly by staining after polyacrylamide gel electrophoresis. The analogue protein was characterised by transforming the mini-cell forming strain E. coli DS410 with the expression plasmid as described in D. Plasmid genes are expressed selectively in minicells allowing the proteins encoded by those genes to be radiolabelled selectively. Thus minicells were labelled with $^{35}$S-methionine and the interferon analogue was characterised by polyacrylamide gel electrophoresis in the presence of SDS run under reducing conditions and revealed by fluorography.

## 2. **Purification by antibody affinity chromatography**

### a) **on NK-2 Sepharose**.

Clarified supernatant (33 ml.) containing the interferon analogue was prepared as hereinbefore described and was applied at a flow rate of about 10 ml./hr. to a column (200 $\mu$l. bed volume) of NK2-sepharose monoclonal antibody immuno-adsorbent support (Celltech Ltd, Slough, UK) equilibrated at room temperature in phosphate buffered saline (PBS) as recommended by the manufacturer. After all the sample was loaded the column was washed with PBS (2 x 1 ml; 1 x 40 ml) and the interferon analogue eluted with 0.1M citric acid - 0.3M NaCl (pH2). The fraction (400 $\mu$l.) containing the analogue was adjusted to pH6 by addition of 450 $\mu$l. of 0.25M mixed phosphate buffer, pH 7.5 (0.25M NaH$_2$PO$_4$ -0.25M Na$_2$HPO$_4$, 1:5). This solution was diluted five-fold and twenty-fold with RPMI-1640 medium containing 10% foetal calf serum, 2mM glutamine, 2mM sodium pyruvate, 1% vitamins, 50 $\mu$g. ml$^{-1}$ gentamycin and 5 x 10$^{-5}$M 2-mercaptoethanol to give samples ready for bioprofiling. These samples were divided into multiple aliquots and stored at -20°; a new aliquot was used for each assay.

The purification was monitored by antiviral and NK2-IRMA assays and the final product was characterised by polyacrylamide gel electrophoresis in the presence of SDS under reducing conditions.

### b. **on YOK-Sepharose**

Clarified supernatant (33 ml.) containing the interferon analogue was prepared as hereinbefore described and was applied at a flow rate of about 10 ml./hr. to a column (200 $\mu$l. bed volume) of YOK-Sepharose monoclonal antibody immuno-absorbent support (Celltech Ltd, Slough, UK) equilibrated at room temperature in phosphate buffered saline (PBS) as recommended by the manufacturer. After all the sample was loaded the column was washed with PBS (2 x 1 ml; 1 x 40 ml.) and the interferon analogue eluted with 0.1M citric acid - 0.3M NaCl (pH2). The fraction (400 $\mu$l.) containing the analogue was adjusted to pH6 by addition of 450 $\mu$l. of 0.25M mixed phosphate buffer, pH 7.5 (0.25M NaH$_2$PO$_4$ -0.25M Na$_2$HPO$_4$, 1:5). This solution was diluted five-fold and twenty-fold with RPMI-1640 medium containing 10% foetal calf serum, 2mM glutamine, 2mM sodium pyruvate, 1% vitamins, 50 $\mu$g. ml$^{-1}$ gentamycin and 5 x 10$^{-5}$M 2-mercaptoethanol to give samples ready for bioprofiling. These samples were divided into multiple aliquots and stored at -20°C.; a new aliquot was used for each assay.

The purification was monitored by antiviral and NK2-IRMA assays and the final product was characterised by polyacrylamide gel eletrophoresis in the presence of SDS under reducing conditions.

The antiviral and NK2-IRMA titres for each relevant analogue are set out in Table 5 below. A hyphen in the NK2-IRMA column indicates no cross-reactivity. The antiviral and NK2-IRMA titres for the analogues of Examples 27-30 were determined at the clone selection stage.

## TABLE 5

Monoclonal antibody purified analogue profiles

| Example No. | Protein | Profile Sample Titre (U/ml.) | |
|---|---|---|---|
| | | AV (x10$^6$) | NK-.2IRMA (x10$^6$) |
| 1 | endo-Asp$^{43a}$-[Glu$^5$,Asp$^{10}$,Asn$^{11}$] IFN-$\alpha_2$ | 6.3 | 61 |
| 2 | endo-Asp$^{43a}$-[Leu$^{151}$,Arg$^{160,163}$] IFN-$\alpha_2$ | 58.0 | 160 |
| 3 | [γ(2-7)$^{2-7}$] IFN-$\alpha_2$ | 1 | 150 |
| 4 | [γ(98-114)$^{98-114}$] IFN-$\alpha_2$ | 6.1 | − |
| 5 | [Met$^{100}$,γ(101-114)$^{101-114}$] IFN-$\alpha_2$ | 0.7 | − |
| 6 | [γ(98-107)$^{98-107}$] IFN-$\alpha_2$ | 11.2 | 130 |
| 7 | [γ(95-101)$^{101-107}$] IFN-$\alpha_2$ | 8.0 | 36 |
| 8 | [γ(2-7),Met$^{100}$,Glu$^{102,103}$,Arg$^{104}$,Gly$^{106}$Asn$^{112}$Ala$^{113}$] IFN-$\alpha_2$ | 3.1 | − |
| 9 | [γ(2-7),γ(101-114)] IFN-$\alpha_2$ | 4.7 | − |
| 10 | [Met$^{100}$, γ(2-7), γ(101-114)] IFN-$\alpha_2$ | 1.0 | − |

Continuation....(2)

**TABLE 5**

| Example No. | Protein | Profile Sample Titre (U/ml.) | |
|---|---|---|---|
| | | AV (x $10^6$) | NK-2IRMA(x$10^6$) |
| 11. | [Leu$^{16,21,59,111,148}$] IFN-$\alpha_2$ | 1.0 | 13 |
| 12. | [Leu$^{16}$] IFN-$\alpha_2$ | 5.5 | 27 |
| 13 | [Leu$^{21}$] IFN-$\alpha_2$ | 5.1 | 64 |
| 14. | [Leu$^{59}$] IFN-$\alpha_2$ | 13.8 | 86 |
| 15. | [Leu$^{111}$] IFN-$\alpha_2$ | 6.2 | 18 |
| 16. | [Ala$^{10,37,44,102,104}$] IFN-$\alpha_2$ | 19.7 | 74 |
| 17. | [Ala$^{137}$] IFN-$\alpha_2$ | 30 | – |
| 18. | [Ser$^{29,138}$] IFN-$\alpha_2$ | 0.18 | 5.5 |
| 19. | [Glu$^{32}$] IFN-$\alpha_2$ | 5 | 41 |
| 20. | [Ala$^{61,64,67}$] IFN-$\alpha_2$ | 0.02 | 0.29 |
| 21. | IFN-$\alpha_2$(12-165) | 0.004 | 0.03 |

32

Continuation....(3)

## TABLE 5

| Example No. | Protein | Profile Sample Titre (U/ml.) | |
|---|---|---|---|
| | | AV (x 10⁶) | NK-2IRMA (x 10⁶) |
| 22. | IFN-α₂(10-165) | 0.003 | 0.01 |
| 23. | Met¹⁰⁰des(101-110)IFN-α₂ | 1.0 | 3.8 |
| 24. | [Ala¹⁰⁻¹⁵]IFN-α₂ | 0.04 | 11 |
| 25. | [Phe¹¹⁵]IFN-α₂ | 0.07 | 0.15 |
| 26. | IFN-α₂(4-155) | 1.6 | 6.4 |
| 27. | [Ala³²]IFN-α₂ | 0.009 | 1.1 |
| 28. | [Ile³⁰]IFN-α₂ | 0.01 | 2.9 |
| 29. | [Asn³²]IFN-α₂ | 0.04 | 0.8 |
| 30. | [Ala²⁸]IFN-α₂ | 0.14 | 2.2 |
| 31. | [Glu⁵, Ser²⁷, Met³¹, Leu⁵⁹]IFN-α₂ | 1.3 | 310 |

**Example 32**

This Example illustrates the preparation of [Leu$^{16,21,59,111,148}$]IFN-α₂. The gene for this protein is hereinafter referred to for convenience as I-I. The procedure described in Examples 1 - 31 was repeated with oligonucleotides 7,8,9,10,24,25,45,46,60 and 61 in Figure 1 replaced by oligonucleotides 7-I2, 8-I2, 9-I3, 10-I3, 24-I4, 25-I4, 45-I5, 46-I5, 60-I6 and 61-I6 (Table 6).

33

TABLE 6

| Oligonucleotide | Sequence (5'-3') | Amino Acid changed | New Amino Acid |
|---|---|---|---|
| 7-12 | CGCACCCTGCTCCTG | 16 | Leu |
| 8-12 | TGGGCCAGCAGGAGC | | |
| 9-13 | CTGGCCCAACTGCGC | | |
| 10-13 | GAGATACGGCGCAGT | 21 | Leu |
| 24-14 | ATCAGTTCGTGCAGT | 59 | Leu |
| 25-14 | GAACTGATTCAACAG | | |
| 45-15 | ACTCCGCTGCTGAAAG | 111 | Leu |
| 46-15 | GAGTCTTCTTTCAGC | | |
| 60-16 | AACGCAGGATTTCAG | 148 | Leu |
| 61-16 | CTGCGTTCCTTCAGC | | |

Fragments A to M were prepared by ligating oligonucleotide mixtures according to the strategy outlined previously. In this Example, fragments B-I 2/3 E-I4, I-I5, J-I5, L-I6, and M-I6 replace fragments B,E,I,J,L and M in Examples 1-31.

Sequences I, II and IIIM were constructed from the fragments shown in Table 7 after phosphorylation of the 5'-hydroxy termini of fragments B to L.

TABLE 7

| Sequence | Fragments used |
|---|---|
| I(I-I) | A-Taq, B-I 2/3, C,D |
| II (I-I) | E-I4, F,G,H |
| IIIM (I-I) | I-I5, J-I5, K, L-I6, M-I6 |

The gene I-I was prepared by ligating sequences I(I-I), II(I-I) and IIIM(I-I) and phosphorylated with T4 polynucleotide kinase.

The gene I-I was cloned as described in Section B of Examples 1-31, and expressed in E.coli JA 221 as described in Section C1, the interferon analogue was extracted by lysis of the bacteria as described in Section D and purified by precipitation and size exclusion chromatography as described in Section E of the aforesaid Examples.

34

The purification was monitored by antiviral and NK2-IRMA assays and the final product characterised by polyacrylamide gel electrophoresis in the presence of SDS under reducing conditions. The titres and recoveries for [Leu[16,21,59,111,148]]IFN-$\alpha_2$ are summarized below:-

TABLE 8

| Sample | Vol. | Antiviral activity | | NK2-IRMA activity | |
|---|---|---|---|---|---|
| | | units/ml. | total units | units/ml. | total units |
| lysate supernatant | 40 | 1.3x10⁴ | 5.2x10⁵ | 4.0x10⁵ | 1.6x10⁷ |
| extracted protein pellet | 21 | N/D | --- | 3.7x10⁵ | 7.8x10⁶ |
| final sterile filtrate | 19 | 2.3x10⁴ | 4.4x10⁵ | 3.6x10⁵ | 6.8x10⁶ |
| recovery | --- | | 85% | | 43% |

## Example 33

### Preparation of [Ala[10−15]]IFN-$\alpha_2$

#### A. Oligonucleotide synthesis

The procedure described in Examples 1-31 was repeated using as the new oligonucleotide sequences 5-A11, 6-A11 and 7-A11 instead of the oligonucleotides 5,6 and 7 respectively. The sequences of 5-A11, 6-A11 and 7-A11 are as follows:-

| oligonucleotide | Sequence (5'-3') |
|---|---|
| 5-A11 | AGCCTGGCTGCAGCT |
| 6-A11 | GCAGCAGCAGCTGCAG |
| 7-A11 | GCTGCTGCGATGCTG |

Fragments A-taq to D were prepared by ligating appropriate oligonucleotide mixtures. In this example, fragment A-A11 replaces A-Taq and B-A11 replaces B. A-A11 comprises oligonucleotides 1 Taq, 2-Taq, 3,4 and 5-A11 and B-A11 comprises oligonucleotides 6-A11, 7-A11, 8, 9 and 10. Ligation of fragments A-A11, B-A11, C and D generates a DNA sequence equivalent to sequence I in Examples 1-31 and includes the Bst E II/Eca I restriction site. Following isolation of the A-D fragment from a polyacrylamide gel it was ethanol precipitated and resuspended in water (37.75 $\mu$l.). Bovine serum albumin (5 $\mu$g. in 5 $\mu$l), Bst E II (3 units in 1 $\mu$l.), a buffer (0.6M Tris HCl pH 7.4, 0.6 M MgCl$_2$ and 0.6M mercaptoethanol) (5 $\mu$l) and 2M NaCl (1.25 $\mu$l.) were added and the mixture incubated at 37°C. for 16 hours. The restriction digests were stopped by addition of 3M NaCl, pH 5.6 (20 $\mu$l.) and water (130 $\mu$l.) and the DNA precipitated with ethanol then fractionated in a 10% polyacrylamide non-denaturing gel. The most-slowly migrating band was eluted from the gel, ethanol precipitated and 5' phosphorylated to give the Cla-Bst E fragment.

## B. Cloning of the [Ala$^{10-15}$]IFN-$\alpha_2$ gene

The vector for cloning the [Ala$^{10-15}$]IFN-$\alpha_2$ gene consisted of the large ClaI-SalI fragment of the plasmid pSTP1 (see examples 1-31) ligated to the small BstEII-SalI interferon gene fragment of the vector pIFS1205 (Edge et al., Nucleic Acids Research, Volume 11 (1983) p 6419). Fragments were isolated as for Examples 1-31 and ligated together prior to reisolation from an agarose gel as above. The DNA concentration was adjusted to 10 $\mu$g./ml. and mixed with a sample of the synthetic [Ala$^{10-15}$] IFN- $\alpha_2$ gene fragment. The mixture was dried and taken up in 30 $\mu$l. of a mixture containing 65 $\mu$l. 10 x ligase buffer, 65 $\mu$l. 1 mg./ml. BSA, 510 $\mu$l. water and 10 $\mu$l. T4 DNA ligase (Boehringer, 1.8 units/$\mu$l.). Incubations were for 1 hour at 4°C. and overnight at 12°C. Ligations were stopped by heating to 65°C. for 15 minutes. 6 $\mu$l. of the ligation mixes were used directly to transform the strain MM294 by the method described by Hanahan (Jour. of Mol. Biol 166 (1983)p.557). Screening for [Ala$^{10-15}$]IFN-$\alpha_2$ gene recombinants by colony hybridisation was as described for Examples 1-31 using nick translated probes for small subfragments as described in Examples 1-31.

In addition, a colony hybridisation analysis was performed with oligonucleotide probes. For screening for [Ala$^{10-15}$]IFN-$\alpha_2$ gene recombinants, the 16 base long oligonucleotide 6-A11 was used. 5 x 10$^{-4}$ 0D units of oligonucleotide was dried in vacuo and taken up in 20 $\mu$l. of a mixture containing 25 $\mu$l. $^{32}$p ATP (10mCi/ml, 5000 ci/mmol, New England Nuclear), 50 $\mu$l. 10 10 x kinase buffer (0.5M Tris pH9, 10mM EDTA), 50 $\mu$l 1mM spermidine, 50 $\mu$l. 0.2M DTT, 50 $\mu$l. 100mM MgCl$_2$ 5 $\mu$l. T4 polynucleotide kinase (2 units/$\mu$l., New England Biolabs) and 270 $\mu$l. water. The labelled oligonucleotide was diluted into 10 ml. 6 x SSC for hybridisation. The hybridisation temperature used was guided by the formula 4(G/C) + 2(A/T)°C. where G/C and A/T denote the number of guanine/cytosine or adenine/thymine nucleotides in the oligonucleotide. Thus, for the probe 6-A11 G/C = 11, A/T = 5), the formula yields the result 54°C., the hybridisation actually being performed at 56°C. for at least 1 hour and filters were subsequently air dried for autoradiography. [Ala$^{10-15}$]IFN-$\alpha_2$ recombinants were identified by positive hybridisation signals. The integrity of the [Ala$^{10-15}$]IFN-$\alpha_2$ analogue gene was checked by Hpa II restriction analysis as for Examples 1 - 31. Lysis of recombinants for biochemical and biological evaluations of interferon were as described in Examples 1 - 31.

The vector containing the [Ala$^{10-15}$]IFN-$\alpha_2$ gene was expressed in E.coli DS410 as described in Section D3 of Examples 1-31, the bacteria lysed as described in Section C and [Ala$^{10-15}$]IFN-$\alpha_2$ purified by antibody affinity chromatography on NK2-Sepharose. The purification was monitored by antiviral and NK2-IRMA assays and the final product characterized by polyacrylamide gel electrophoresis in the presence of SDS under reducing conditions. The titres and recoveries for [Ala$^{10-15}$]IFN-$\alpha_2$ are summarized below:-

**TABLE 9**

| Sample | Vol. | Antiviral Activity units/ml. | Antiviral Activity total units | NK2-IRMA Activity units/ml. | NK2-IRMA Activity total units |
|---|---|---|---|---|---|
| lysate supernatant | 36 | $2.1 \times 10^3$ | $7.6 \times 10^6$ | $4.9 \times 10^5$ | $1.8 \times 10^7$ |
| column eluate pH6 | 0.85 | $3.2 \times 10^4$ | $2.7 \times 10^8$ | $1.1 \times 10^7$ | $9.4 \times 10^6$ |
| recovery | | | 36% | | 52% |

**Example 34**

The procedure of Example 33 was repeated except that the oligonucleotides 17 and 18 were replaced by the oligonucleotides 17-Bst E and 18-BstE:-

37

| Oligonucleotide | Sequence (5'-3') |
|---|---|
| 17-Bst E | TTCCCGCAGGAAGAGTTCG |
| 18-Bst E | GTTACCGAACTCTTCC |

Fragment D was thus replaced by fragment D-Bst E, prepared from oligonucleotides 16, 17-Bst E and 18-Bst E. Ligation of A-taq, B, C-K11 and D-Bst E, followed by phosphorylation of the 5' ends with T4 polynucleotide kinase and ATP and isolation of the product by gel-electrophoresis gives the Cla-BstE fragment.

The use of the modified fragment D ensures that the single-stranded cohesive end for Bst E II/Eca I is present.

**Example 35**

The procedure described in Examples 1 to 31 was repeated in order to prepare the analogue of Example 21 except that oligonucleotides 4 and 5 as detailed below were replaced by alternative sequences which introduced restriction enzyme sites,but retained the protein sequence of the interferon analogue.

Example 49

TABLE 10

| Oligonucleotide Changed | New sequence (5'-3') | Restriction Site Introduced | New small sub-fragment |
|---|---|---|---|
| 4 | | | |
| 5 | | | |
| 4 Sau I | CTAAGGAATGAGTTG | Sau I | |
| 5 Sau I | TCCTTAGGTAGCCGT | | A-Sau I |

Analogues of the aforementioned Examples were tested for antiviral and antiproliferative activity in the following assays:-

(i) Antiviral assay

Antiviral titers were determined in cytopathic effect inhibition assays by a dye uptake method, using the human amnion cell line WISH challenged with encephalomyocarditis virus. The assay was calibrated using the NIH human leukocyte IFN standard, G-023-901-527. An internal standard was employed in every assay.

(ii) Growth inhibition assay

The Daudi cell line was grown in RPMI-1640 medium supplemented with 20mM HEPES and 10% FCS. Cultures containing $5 \times 10^4$ Daudi cells in 100 $\mu$l. of complete medium were incubated with 100 $\mu$l. of medium containing 0 to 1000U. of IFN for 72 hr. at 37°C. Four replicates were used for each sample dilution. [$^3$H]Thymidine (1$\mu$.Ci in 50 $\mu$l.; 5 Ci/mmol) was added, and the cultures were incubated for a further 24 hr. Cultures were subsequently processed using an automated cell harvester. Results were expressed as the percentage inhibition of [$^3$H]-thymidine uptake of test sample compared with a medium control.

The tested analogues were all found to possess antiviral activity and the analogues of Examples 2, 4, 5, 11, 16, 18 and 26 were in particular found to possess a ratio of antiviral to antiproliferative activity (AV/AP) substantially better than the AV/AP of 3 reported in the literature for natural human IFN-$\alpha_2$.

**Claims**

1. A polypeptide of the formula I:-

$$X^1-X^2-X^3-X^4-X^5-X^6-X^7-X^8-X^9-X^{10}-X^{11}-X^{12}-X^{13}-$$
$$-X^{14}-X^{15}-X^{16}-Leu-Leu-Ala-Gln-X^{21}-X^{22}-X^{23}-Ile-Ser-X^{26}-$$
$$-X^{27}-X^{28}-X^{29}-X^{30}-X^{31}-X^{32}-Arg-X^{34}-Asp-Phe-X^{37}-X^{38}-Pro-$$
$$-Gln-Glu-Glu-Phe-X^{43a}-X^{44}-Asn-Gln-Phe-Gln-Lys-X^{50}-X^{51}-$$
$$-X^{52}-Ile-X^{54}-Val-Leu-His-Glu-X^{59}-Ile-X^{61}-Gln-X^{63}-$$
$$-X^{64}-Asn-Leu-X^{67}-X^{68}-Thr-X^{70}-Asp-Ser-Ser-Ala-Ala-Trp-$$
$$-X^{77}-X^{78}-X^{79}-Leu-Leu-X^{82}-Lys-Phe-X^{85}-Thr-Glu-Leu-Tyr-$$
$$-Gln-Gln-Leu-Asn-X^{94}-Leu-Glu-Ala-X^{98}-X^{99}-X^{100}-X^{101}-$$
$$-X^{102}-X^{103}-X^{104}-X^{105}-X^{106}-X^{107}-X^{108}-X^{109}-X^{110}-X^{111}-$$
$$-X^{112}-X^{113}-X^{114}-X^{115}-Ile-Leu-Ala-Val-X^{120}-Lys-Tyr-Phe-$$
$$-X^{124}-Arg-Ile-Thr-Leu-Tyr-Leu-X^{131}-Glu-Lys-Lys-Tyr-$$
$$-Ser-X^{137}-X^{138}-Ala-Trp-Glu-Val-Val-Arg-Ala-Glu-$$
$$-Ile-X^{148}-Arg-Ser-X^{151}-Ser-X^{153}-Ser-X^{155}-X^{156}-X^{157}-$$
$$-X^{158}-X^{159}-X^{160}-X^{161}-X^{162}-X^{163}-X^{164}-X^{165}$$

in which $X^1,X^2,X^3,X^4,X^5,X^5,X^7,X^5,X^5,X^{10},X^{11},X^{155},X^{156}, X^{157},X^{158},X^{159},X^{160},X^{161},X^{162},X^{163},X^{164}$ and $X^{165}$ may each represent a naturally occurring alpha-amino acid and $X^{12}$ represents Arg or Ala, $X^{13}$ represents Arg or Ala, $X^{14}$ represents Thr or Ala, $X^{15}$ represents Leu or Ala, $X^{16}$ represents Met, Leu or Ile, $X^{21}$ represents Met or Leu, $X^{22}$ represents Ser, Arg or Gly and $X^{23}$ represents Arg or Lys, $X^{26}$ represents Leu or Pro, $X^{27}$ represents Ser or Phe, $X^{28}$ represents Ser or Ala $X^{29}$ represents Ser or Cys, $X^{30}$ represents Ile or Leu $X^{31}$ represents Met or Lys, $X^{32}$ represents Ala, Asn, Asp or Glu and $X^{34}$ represents His or Pro, $X^{37}$ represents Gly or Ala, $X^{38}$ represents Phe or Leu, $X^{43a}$ represents a single bond or Asp, $X^{44}$ represents Gly or Ala, $X^{50}$ represents Ala or Thr, $X^{51}$ represents Glu, Pro or Gln $X^{52}$ represents Ala or Thr, and $X^{54}$ represents Ser or Pro, $X^{59}$ represents Leu or Met, $X^{51}$ represents Gln or Ala, $X^{53}$ represents Ile or Thr, $X^{54}$ represents Phe or Ala, $X^{57}$ represents Phe or Ala, $X^{58}$ represents Thr or Ser, $X^{70}$ represents Lys or Glu, $X^{77}$ represents Asp or Glu, $X^{78}$ represents Glu or Gln, $X^{79}$ represents Asp, Thr or Ser, $X^{52}$ represents Asp or Glu $X^{55}$ represents Cys, Tyr or Ser, $X^{54}$ represents Asp or Asn, $X^{58}$ represents Cys or Leu, $X^{59}$ represents Val or Thr, $X^{100}$ represents Met, Ile or Asn, either $X^{101}$ represents Gln, Tyr or Phe, $X^{102}$ represents Glu, Gly, Ser or Ala, $X^{103}$ represents Glu, Val or Lys, $X^{104}$ represents Arg, Gly, Thr, Leu or Ala, $X^{105}$ represents Val, Asp, Met or Thr, $X^{106}$ represents

Gly, Thr, Glu, Leu or Asn, $X^{107}$ represents Glu, Asn or Tyr, $X^{108}$ represents Thr or Val, $X^{109}$ represents Pro or Gln, $X^{110}$ represents Leu or Arg and $X^{111}$ represents Met, Lys or Leu,

or $-X^{101}-X^{102}-X^{103}-X^{104}-X^{105}-X^{106}-X^{107}-X^{108}-X^{109}-X^{110}-X^{111}-$ represents Met, Lys or Leu, $-X^{112}$ represents Asn, Lys or Ala, $X^{113}$ represents Ala, Glu or Ile, $X^{114}$ represents Asp or His, $X^{115}$ represents Ser or Phe, $X^{120}$ represents Lys or Arg, $X^{124}$ represents Arg or Gln, $X^{131}$ represents Thr or Lys, $X^{137}$ represents Pro or Ala, $X^{138}$ represents Cys or Ser, $X^{148}$ represents Met or Leu, $X^{151}$ represents Leu or Phe and $X^{153}$ represents Leu or Phe, the amino acids being selected such that an amino acid is present in at least one position selected from alpha$_2$ positions 12-16, 21, 28,29, 30, 32, 37, 44, 61, 64, 67, 98-115, 137, 138 and 148 which differs from the amino acid in the corresponding position of IFN-alpha$_2$, IFN-alpha$_1$ and IFN-alphaI and/or selected such that an amino acid is absent from at least one position selected from alpha$_2$ positions 101-110;

or the amino acids being selected such that an amino acid is present in at least one position selected from alpha$_2$ positions 27, 31, 59, 151, 160 and 163 which is the same as the amino acid in the corresponding position of IFN-alpha$_1$, the amino acids at alpha$_2$ positions other than positions 1 to 11, 27, 31,43a, 59, 100, 102-104, 106, 112, 113, 151 and 156 to 165 being the same as the amino acids in the corresponding positions of IFN-alpha$_2$) or a $N_{1-11}$ and/or $C_{1-11}$ truncated analogue thereof and the $N_{3-11}$ truncated analogues of IFN-alpha$_2$, which may if desired be $C_{1-11}$ truncated, with the proviso that a $N_3$-truncated analogue of IFN-alpha$_2$ is also $C_{7-11}$ truncated.

2. A polypeptide as claimed in claim 1 wherein $X^1$ represents Cys, $X^2$ represents Asp or Trp, $X^3$ represents Leu or Cys, $X^4$ represents Pro or Gln, $X^5$ represents Glu, Gln or Asp, $X^5$ represents Thr or Pro, $X^7$ represents His or Tyr, $X^5$ represents Ser, $X^5$ represents Leu, $X^{10}$ represents Asp, Gly or Ala, $X^{11}$ represents Asn, Ser or Ala, $X^{155}$ represents Thr, $X^{155}$ represents Thr, $X^{156}$ represents Asn, $X^{157}$ represents Leu, $X^{158}$ represents Gln, $X^{159}$ represents Glu or Lys, $X^{160}$ represents Arg, Ser or Ile, $X^{161}$ represents Leu, $X^{162}$ represents Arg, $X^{163}$ represents Arg or Ser, $X^{164}$ represents Lys and $X^{165}$ represents Glu or Asp.

3. A polypeptide as claimed in claim 1 which is $N_{1-3}$ and/or $C_{1-10}$ truncated.

4. A polypeptide as claimed in claim 1 of the formula II:-

```
Cys-Asp-Leu-Pro-X⁵ᵃ-Thr-His-Ser-Leu-X¹⁰ᵃ-X¹¹ᵃ-Arg-Arg-
    Thr-Leu-Met-Leu-Leu-Ala-Gln-Met-Arg-Lys-Ile-Ser-Leu-
    X²⁷ᵃ-Ser-Cys-Leu-X³¹ᵃ-Asp-Arg-His-Asp-Phe-Gly-Phe-Pro-
    Gln-Glu-Glu-Phe-X⁴³ᵃ-Gly-Asn-Gln-Phe-Gln-Lys-Ala-Glu-
    Tyr-Ile-Pro-Val-Leu-His-Glu-X⁵⁹ᵃ-Ile-Gln-Gln-Ile-Phe-
    Asn-Leu-Phe-Ser-Thr-Lys-Asp-Ser-Ser-Ala-Ala-Trp-Asp-
    Glu-Thr-Leu-Leu-Asp-Lys-Phe-Tyr-Thr-Glu-Leu-Tyr-Gln-
    Gln-Leu-Asn-Asp-Leu-Glu-Ala-Cys-Val-X¹⁰⁰ᵃ-Gln-
    X¹⁰²ᵃ-X¹⁰³ᵃ-X¹⁰⁴ᵃ-Val-X¹⁰⁶ᵃ-Glu- Thr-Pro-Leu-Met-
    X¹¹²ᵃ-X¹¹³ᵃ-Asp-Ser-Ile-Leu-Ala-Val-Arg-Lys-Tyr-Phe-
    Gln-Arg-Ile-Thr-Leu-Tyr-Leu-Lys-Glu-Lys-Lys-Tyr-Ser-
    Pro-Cys-Ala-Trp-Glu-Val-Val-Arg-Ala-Gln-Ile-Met-Arg-
    Ser-X¹⁵¹ᵃ-Ser-Leu-Ser-Thr-Asn-Leu-Gln-Glu-X¹⁶⁰ᵃ-
    Leu-Arg-X¹⁶³ᵃ-Lys-Glu
```

(in which X⁵ᵃ represents Glu or Gln, X¹⁰ᵃ represents Asp or Gly, X¹¹ᵃ represents Asn or Ser, X²⁷ᵃ represents Phe or Ser, X²¹ᵃ represents Lys or Met, X⁴³ᵃ represents Asp or a single bond, X⁵⁹ᵃ represents Met or Leu, X¹⁰⁰ᵃ represents Met or Ile, X¹⁰²ᵃ represents Glu or Gly, X¹⁰³ᵃ represents Glu or Val, X¹⁰⁴ᵃ represents Arg or Gly, X¹⁰⁶ᵃ represents Gly or Thr, X¹¹²ᵃ represents Asn or Lys, X¹¹³ᵃ represents Ala or Glu, X¹⁵¹ᵃ represents Leu or Phe, X¹⁶⁰ᵃ represents Arg or Ser and X¹⁶³ᵃ represents Arg or Ser with the proviso that the amino acids are selected such that at least one of X²⁷ᵃ, X²¹ᵃ, X⁵⁹ᵃ, X¹⁵¹ᵃ, X¹⁶⁰ᵃ and X¹⁶³ᵃ, represents the same amino acid as that in the corresponding position of IFN-alpha₁).

5. A polypeptide as claimed in claim 1 of the formula II as defined in claim 4 in which X⁴³ᵃ represents Asp, X¹⁰⁰ᵃ represents Ile, X¹⁰²ᵃ represents Gly, X¹⁰³ᵃ represents Val, X¹⁰⁴ᵃ represents Gly, X¹⁰⁶ᵃ represents Thr, X¹¹²ᵃ represents Lys and X¹¹³ᵃ represents Glu with the proviso that at least one of X⁵ᵃ, X¹⁰ᵃ, X¹¹ᵃ, X¹⁵¹ᵃ, X¹⁶⁰ᵃ and X¹⁶³ᵃ represents an amino acid which differs from the amino acid in the corresponding position of IFN-alpha₂.

6. A polypeptide as claimed in claim 1 wherein X¹ represents Cys, X² represents Asp or Trp, X³ represents Leu or Cys, X⁴ represents Pro or Glu, X⁵ represents Gln or Asp, X⁵ represents Thr or Pro, X⁷ represents His or Tyr, X⁵ represents Ser, X⁵ represents Leu, X¹⁰ represents Gly or Ala, X¹¹ represents Ser or Ala, $X^{16}$ represents Leu or Met, $X^{22}$ represents Arg, $X^{23}$ represents Lys, $X^{26}$ represents Leu, $X^{27}$ represents Phe, $X^{31}$ represents Lys, $X^{34}$ represents His, $X^{38}$ represents Phe, $X^{43a}$ represents a single bond, $X^{50}$ represents Ala, $X^{51}$ represents Glu, $X^{52}$ represents Thr, $X^{54}$ represents Pro, $X^{53}$ represents Ile, $X^{58}$ represents Ser, $X^{70}$ represents Lys $X^{77}$ represents Asp, $X^{78}$ represents Glu, $X^{79}$ represents Thr, $X^{52}$ represents Asp, $X^{55}$ represents Tyr, $X^{54}$ represents Asp, $X^{105}$ represents Val, Asp or Thr, $X^{106}$ represents Thr, Leu or Asn, $X^{120}$ represents Arg, $X^{124}$ represents Gln, $X^{131}$ represents Lys, $X^{151}$ represents Phe, $X^{153}$ represents Leu, $X^{156}$ represents Asn, $X^{157}$ represents Leu, $X^{158}$ represents Gln, $X^{159}$ represents Glu, $X^{160}$ represents Ser, $X^{161}$ represents Leu, $X^{162}$ represents Arg, $X^{163}$ represents Ser, $X^{164}$ represents Lys and $X^{165}$ represents Glu.

7. A polypeptide as claimed in claim 1 of the formula:-

$$
\begin{aligned}
&\text{Cys } X^2X^3X^4X^5X^6X^7\text{-Ser-Leu-}X^{10c}\text{-Ser-Arg-Arg-Thr-Leu-Met}\\
&\text{Leu-Leu-Ala-Gln-Met-Arg-Lys-Ile-Ser-Leu-Phe-Ser-Cys}\\
&\text{Leu-Lys-Asp-Arg-His-Asp-Phe-}X^{37}\text{-Phe-Pro-Gln-Glu-Glu}\\
&\text{Phe-}X^{44}\text{-Asn-Gln-Phe-Gln-Lys-Ala-Glu-Thr}\\
&\text{Ile-Pro-Val-Leu-His-Glu-}X^{59}\text{-Ile-Gln-Gln-Ile-Phe-Asn}\\
&\text{Leu-Phe-Ser-Thr- Lys-Asp-Ser-Ser-Ala-Ala-Trp-Asp-Glu}\\
&\text{Thr-Leu-Leu-Asp-Lys-Phe-Tyr-Thr-Glu-Leu-Tyr-Gln-Gln}\\
&\text{Leu-Asn- Asp-Leu-Glu-Ala-}X^{98c}\text{-}X^{99c}\text{-}X^{100c}\text{-}X^{101c}\\
&X^{102c}\text{-}X^{103c}\text{-}X^{104c}\text{-}X^{105c}\text{-}X^{106c}\text{-}X^{107c}\text{-}X^{108}\text{-}X^{109}\text{-}X^{110}\\
&X^{111c}\text{-}X^{112c}\text{-}X^{113c}\text{-}X^{114}\text{-Ser-Ile-Leu-Ala-Val-Arg-Lys}\\
&\text{Tyr-Phe -Gln-Arg-Ile-Thr-Leu-Tyr-Leu-Lys-Glu-Lys-Lys}\\
&\text{Tyr-Ser-Pro-Cys-Ala-Trp-Glu-Val-Val-Arg-Ala-Glu-Ile}\\
&\text{Met- Arg-Ser-Phe-Ser-Leu-Ser-Thr-Asn-Leu-Gln-Glu-Ser}\\
&\text{Leu-Arg-Ser-Lys-Glu}
\end{aligned}
$$

(wherein $X^2$ represents Asp or Trp, $X^3$ represents Leu or Cys, $X^4$ represents Pro or Gln, $X^5$ represents Gln or Asp, $X^5$ represents Thr or Pro, $X^7$ represents His or Tyr,

$X^{10c}$ represents Gly or Ala, $X^{37}$ represents Gly or Ala, $X^{44}$ represents Gly or Ala, $X^{59}$ represents Met or Leu, $X^{98c}$ represents Cys or Leu, $X^{99c}$ represents Val or Thr, $X^{100c}$ represents Ile, Met or Asn, $X^{101c}$ represents Gln or Tyr, $X^{102c}$ represents Gly, Ala or Ser, $X^{103c}$ represents Val, $X^{104c}$ represents Gly, Ala or Thr, $X^{105c}$ represents Val or Asp, $X^{106c}$ represents Thr or Leu, $X^{107c}$ represents Glu or Asn, $X^{108}$ represents Thr or Val, $X^{109}$ represents Pro or Gln, $X^{110}$ represents Leu or Arg, $X^{111c}$ represents Met or Lys, $X^{112c}$ represents Lys or Ala, $X^{113c}$ represents Glu or Ile, and $X^{114}$ represents Asp or His) and corresponding polypeptides in which Cys at positions 29 and 138 is replaced by Ser, the amino acids being selected such that at least in one of positions 2-7,10,37,44,59 and 99-114 an amino acid is present which differs from the amino acid in the corresponding position of IFN-alpha$_2$.

8. A polypeptide as claimed in claim 1 in which $-X^{58}\text{-}X^{59}\text{-}X^{100}\text{-}X^{101}\text{-}X^{102}\text{-}X^{103}\text{-}X^{104}\text{-}X^{105}\text{-}X^{106}\text{-}X^{107}\text{-}X^{108}\text{-}X^{109}\text{-}X^{110}\text{-}X^{111}\text{-}X^{112}\text{-}X^{113}\text{-}X^{114}$- represents the moiety -Leu-Thr-Asn-Tyr-Ser-Val-Thr-Asp-Leu-Asn-Val-Gln-Arg-Lys-Ala-Ile-His-.

44

**9.** A polypeptide as claimed in claim 1 in which at least one of $X^{10}$, $X^{37}$, $X^{44}$, $X^{102}$ and $X^{104}$ represents alanine.

**10.** A polypeptide as claimed in claim 1 in which $X^{59}$ represents leucine.

**11.** A polypeptide as claimed in claim 1 selected from the group consisting of:-

$$\text{IFN-alpha}_2(4\text{-}155)$$
$$\text{endo-Asp}^{43a}\text{-}[\text{Leu}^{151},\text{Arg}^{160,163}]\text{IFN-alpha}_2$$
$$[\text{gamma}(98\text{-}114)^{98\text{-}114}]\text{IFN-alpha}_2$$
$$[\text{Met}^{100},\ \text{gamma}(101\text{-}114)^{101\text{-}114}]\text{IFN-alpha}_2$$
$$[\text{gamma}(2\text{-}7),\ \text{gamma}(98\text{-}114)]\text{IFN-alpha}_2$$
$$[\text{Leu}^{59}]\text{IFN-alpha}_2$$
$$[\text{Ala}^{10,37,44,102,104}]\text{IFN-alpha}_2$$
$$[\text{Ser}^{29,138}]\text{IFN-alpha}_2$$

**12.** A process for producing a polypeptide as defined in claim 1 which comprises culturing a microorganism, the microorganism having been transformed with a replicable plasmidic expression vehicle comprising genetic material coding for the said polypeptide whereby to effect expression of the said polypeptide and recovering the said polypeptide thereby expressed.

**13.** A transformant microorganism capable of expressing a polypeptide as defined in claim 1, the said microorganism comprising a replicable plasmidic expression vehicle, which vehicle comprises genetic material coding for the said polypeptide.

**14.** A process for the preparation of a transformant microorganism as defined in claim 13 which comprises transforming a microorganism by the insertion therein of a replicable plasmidic expression vehicle, which vehicle comprises genetic material coding for a polypeptide as defined in claim 1.

**15.** A replicable plasmidic expression vehicle capable, in a transformant microorganism, of expressing a polypeptide as defined in claim 1.

**16.** A process for the preparation of a replicable plasmidic expression vehicle as defined in claim 15 which comprises inserting a gene coding for a polypeptide as defined in claim 1 into a vector therefor at an appropriate insertion site.

**17.** A process for the preparation of a replicable plasmidic expression vehicle as defined in claim 15 which comprises inserting a nucleotide sequence coding for a portion of the polypeptide as defined in claim 1 into a vector comprising a promoter sequence and a nucleotide sequence(s) coding for the remainder of the polypeptide as detined in claim 1 at an appropriate insertion site whereby a replicable plasmidic expression vehicle is obtained capable of directing the synthesis of the said polypeptide in a transformant microorganism.

**18.** A DNA sequence that encodes for a polypeptide as defined in claim 1.

**19.** A portable expression unit comprising the EcoRI-SalI segment of the plasmid pSTP1 in which a DNA sequence as defined in claim 18 is present between the ClaI and SalI restriction sites of the said segment.

**20.** A pharmaceutical composition comprising as active ingredient at least one polypeptide as defined in claim 1 in association with a pharmaceutically acceptable carrier or excipient.

**Revendications**

1. Polypeptide de formule I :

$$X^1-X^2-X^3-X^4-X^5-X^6-X^7-X^8-X^9-X^{10}-X^{11}-X^{12}-X^{13}-$$
$$-X^{14}-X^{15}-X^{16}-Leu-Leu-Ala-Gln-X^{21}-X^{22}-X^{23}-Ile-Ser-X^{26}-$$
$$-X^{27}-X^{28}-X^{29}-X^{30}-X^{31}-X^{32}-Arg-X^{34}-Asp-Phe-X^{37}-X^{38}-Pro-$$
$$-Gln-Glu-Glu-Phe-X^{43a}-X^{44}-Asn-Gln-Phe-Gln-Lys-X^{50}-X^{51}-$$
$$-X^{52}-Ile-X^{54}-Val-Leu-His-Glu-X^{59}-Ile-X^{61}-Gln-X^{63}-$$
$$-X^{64}-Asn-Leu-X^{67}-X^{68}-Thr-X^{70}-Asp-Ser-Ser-Ala-Ala-Trp-$$
$$-X^{77}-X^{78}-X^{79}-Leu-Leu-X^{82}-Lys-Phe-X^{85}-Thr-Glu-Leu-Tyr-$$
$$-Gln-Gln-Leu-Asn-X^{94}-Leu-Glu-Ala-X^{98}-X^{99}-X^{100}-X^{101}-$$
$$-X^{102}-X^{103}-X^{104}-X^{105}-X^{106}-X^{107}-X^{108}-X^{109}-X^{110}-X^{111}-$$
$$-X^{112}-X^{113}-X^{114}-X^{115}-Ile-Leu-Ala-Val-X^{120}-Lys-Tyr-Phe-$$
$$-X^{124}-Arg-Ile-Thr-Leu-Tyr-Leu-X^{131}-Glu-Lys-Lys-Tyr-$$
$$-Ser-X^{137}-X^{138}-Ala-Trp-Glu-Val-Val-Arg-Ala-Glu-$$
$$-Ile-X^{148}-Arg-Ser-X^{151}-Ser-X^{153}-Ser-X^{155}-X^{156}-X^{157}-$$
$$-X^{158}-X^{159}-X^{160}-X^{161}-X^{162}-X^{163}-X^{164}-X^{165}$$

dans laquelle $X^1$, $X^2$, $X^3$, $X^4$, $X^5$, $X^5$, $X^7$, $X^5$, $X^5$, $X^{10}$, $X^{11}$, $X^{155}$, $X^{156}$, $X^{157}$, $X^{158}$, $X^{159}$, $X^{160}$, $X^{161}$, $X^{163}$, $X^{164}$ et $X^{165}$ peuvent représenter chacun un alpha-amino-acide naturel et $X^{12}$ représente Arg ou Ala, $X^{13}$ représente Arg ou Ala, $X^{14}$ représente Thr ou Ala, $X^{15}$ représente Leu ou Ala, $X^{16}$ représente Met, Leu ou Ile, $X^{21}$ représente Met ou Leu, $X^{22}$ représente Ser, Arg ou Gly et $X^{23}$ représente Arg ou Lys, $X^{26}$ représente Leu ou Pro, $X^{27}$ représente Ser ou Phe, $X^{28}$ représente Ser ou Ala, $X^{29}$ représente Ser ou Cys, $X^{30}$ représente Ile ou Leu, $X^{31}$ représente Met ou Lys, $X^{32}$ représente Ala, Asn, Asp ou Glu et $X^{34}$ représente His ou Pro, $X^{37}$ représente Gly ou Ala, $X^{38}$ représente Phe ou Leu, $X^{43a}$ représente une liaison simple ou Asp, $X^{44}$ représente Gly ou Ala, $X^{50}$ représente Ala ou Thr, $X^{51}$ représente Glu, Pro ou Gln, $X^{52}$ représente Ala ou Thr et $X^{54}$ représente Ser ou Pro, $X^{59}$ représente Leu ou Met, $X^{51}$ représente Gln ou Ala, $X^{53}$ représente Ile ou Thr, $X^{54}$ représente Phe ou Ala, $X^{57}$ représente Phe ou Ala, $X^{58}$ représente Thr ou Ser, $X^{70}$ représente Lys ou Glu, $X^{77}$ représente Asp ou Glu, $X^{78}$ représente Glu ou Gln, $X^{79}$ représente Asp, Thr ou Ser, $X^{52}$ représente Asp ou Glu, $X^{55}$ représente Cys, Tyr ou Ser, $X^{54}$ représente Asp ou Asn, $X^{58}$ représente Cys ou Leu, $X^{59}$ représente Val ou Thr, $X^{100}$ représente Met, Ile ou Asn, $X^{101}$ représente Gln, Tyr ou Phe, $X^{102}$ représente Glu, Gly, Ser ou Ala, $X^{103}$ représente Glu, Val ou Lys, $X^{104}$ représente Arg, Gly, Thr, Leu ou Ala, $X^{105}$ représente Val, Asp, Met ou

46

Thr, $X^{106}$ représente Gly, Thr, Glu, Leu ou Asn, $X^{107}$ représente Glu, Asn ou Tyr, $X^{108}$ représente Thr ou Val, $X^{109}$ représente Pro ou Gln, $X^{110}$ représente Leu ou Arg et $X^{111}$ représente Met, Lys ou Leu, ou bien $-X^{101}-X^{102}-X^{103}-X^{104}-X^{105}-X^{106}-X^{107}-X^{108}-X^{109}-X^{110}-X^{111}-$ représente Met, Lys ou Leu, $-X^{112}$ représente Asn, Lys ou Ala, $X^{113}$ représente Ala, Glu ou Ile, $X^{114}$ représente Asp ou His, $X^{115}$ représente Ser ou Phe, $X^{120}$ représente Lys ou Arg, $X^{124}$ représente Arg ou Gln, $X^{131}$ représente Thr ou Lys, $X^{137}$ représente Pro ou Ala, $X^{138}$ représente Cys ou Ser, $X^{148}$ représente Met ou Leu, $X^{151}$ représente Leu ou Phe et $X^{153}$ représente Leu ou Phe, les amino-acides étant choisis de sorte que soit présent un amino-acide dans au moins une position choisie entre les positions $alpha_2$ 12-16, 21, 28, 29, 30, 32, 37, 44, 61, 64, 67, 98-115, 137, 138 et 148 qui diffère de l'amino-acide dans la position correspondante de IFN-$alpha_2$, IFN-$alpha_1$ et IFN-$alphaI$, et/ou étant choisis de sorte qu'un amino-acide soit absent d'au moins une position choisie entre les positions $alpha_2$ 101-110 ;

ou bien les amino-acides étant choisis, de sorte que soit présent un amino-acide dans au moins une position choisie entre les positions $alpha_2$ 27, 31, 59, 151, 160 et 163 qui soit identique à l'amino-acide dans la position correspondante de IFN-$alpha_1$, les amino-acides en positions $alpha_2$ autres que les positions 1 à 11, 27, 31, 43a, 59, 100, 102-104, 106, 112, 113, 151 et 156 à 165 étant identiques aux amino-acides dans les positions correspondantes de IFN-$alpha_2$ ou d'un de ses analogues tronqués $N_{1-11}$ et/ou $C_{1-11}$ et les analogues tronqués en $N_{3-11}$ de IFN-$alpha_2$, qui peuvent être, le cas échéant, tronqués en $C_{1-11}$, sous réserve qu'un analogue tronqué en $N_3$ de IFN-$alpha_2$ soit également tronqué en $C_{7-11}$.

2. Polypeptide suivant la revendication 1, dans lequel $X^1$ représente Cys, $X^2$ représente Asp ou Trp, $X^3$ représente Leu ou Cys, $X^4$ représente Pro ou Gln, $X^5$ représente Glu, Gln ou Asp, $X^5$ représente Thr ou Pro, $X^7$ représente His ou Tyr, $X^5$ représente Ser, $X^5$ représenté Leu, $X^{10}$ représente Asp, Gly ou Ala, $X^{11}$ représente Asn, Ser ou Ala, $X^{155}$ représente Thr, $X^{156}$ représente Asn, $X^{157}$ représente Leu, $X^{158}$ représente Gln, $X^{159}$ représente Glu ou $X^{162}$ représente Arg, $X^{163}$ représente Arg ou Ser, $X^{164}$ représente Lys et $X^{165}$ représente Glu ou Asp.

3. Polypeptide suivant la revendication 1, qui est tronqué en $N_{1-3}$ et/ou $C_{1-10}$.

**4.** Polypeptide suivant la revendication 1, de formule II :

$$Cys-Asp-Leu-Pro-X^{5a}-Thr-His-Ser-Leu-X^{10a}-X^{11a}-Arg-Arg$$
$$Thr-Leu-Met-Leu-Leu-Ala-Gln-Met-Arg-Lys-Ile-Ser-Leu$$
$$X^{27a}-Ser-Cys-Leu-X^{31a}-Asp-Arg-His-Asp-Phe-Gly-Phe-Pro$$
$$Gln-Glu-Glu-Phe-X^{43a}-Gly-Asn-Gln-Phe-Gln-Lys-Ala-Glu$$
$$Tyr-Ile-Pro-Val-Leu-His-Glu-X^{59a}-Ile-Gln-Gln-Ile-Phe$$
$$Asn-Leu-Phe-Ser-Thr-Lys-Asp-Ser-Ser-Ala-Ala-Trp-Asp$$
$$Glu-Thr-Leu-Leu-Asp-Lys-Phe-Tyr-Thr-Glu-Leu-Tyr-Gln$$
$$Gln-Leu-Asn-Asp-Leu-Glu-Ala-Cys-Val-X^{100a}-Gln$$
$$X^{102a}-X^{103a}-X^{104a}-Val-X^{106a}-Glu-Thr-Pro-Leu-Met$$
$$X^{112a}-X^{113a}-Asp-Ser-Ile-Leu-Ala-Val-Arg-Lys-Tyr-Phe$$
$$Gln-Arg-Ile-Thr-Leu-Tyr-Leu-Lys-Glu-Lys-Lys-Tyr-Ser$$
$$Pro-Cys-Ala-Trp-Glu-Val-Val-Arg-Ala-Gln-Ile-Met-Arg$$
$$Ser-X^{151a}-Ser-Leu-Ser-Thr-Asn-Leu-Gln-Glu-X^{160a}$$
$$Leu-Arg-X^{163a}-Lys-Glu$$

(dans laquelle $X^{5a}$ représente Glu ou Gln, $X^{10a}$ représente Asp ou Gly, $X^{11a}$ représente Asn ou Ser, $X^{27a}$ représente Phe ou Ser, $X^{21a}$ représente Lys ou Met, $X^{43a}$ représente Asp ou une liaison simple, $X^{59a}$ représente Met ou Leu, $X^{100a}$ représente Met ou Ile, $X^{102a}$ représente Glu ou Gly, $X^{103a}$ représente Glu ou Val, $X^{104a}$ représente Arg ou Gly, $X^{106a}$ représente Gly ou Thr, $X^{112a}$ représente Asn ou Lys, $X^{113a}$ représente Ala ou Glu, $X^{151a}$ représente Leu ou Phe, $X^{160a}$ représente Arg ou Ser et $X^{163a}$ représente Arg ou Ser, sous réserve que les amino-acides soient choisis de sorte qu'au moins l'un de $X^{27a}$, $X^{21a}$, $X^{59a}$, $X^{151a}$, $X^{160a}$ et $X^{163a}$ représente le même amino-acide que celui présent dans la position correspondante de IFN-alpha$_1$).

**5.** Polypeptide suivant la revendication 1, de formule II suivant la revendication 4, dans lequel $X^{43a}$ représente Asp, $X^{100a}$ représente Ile, $X^{102a}$ représente Gly, $X^{103a}$ représente Val, $X^{104a}$ représente Gly, $X^{106a}$ représente Thr, $X^{112a}$ représente Lys et $X^{113a}$ représente Glu, sous réserve qu'au moins l'un de $X^{5a}$, $X^{10a}$, $X^{11a}$, $X^{151a}$, $X^{160a}$ et $X^{163a}$ représente un amino-acide qui diffère de l'amino-acide dans la position correspondante de IFN-alpha$_2$.

**6.** Polypeptide suivant la revendication 1, dans lequel $X^1$ représente Cys, $X^2$ représente Asp ou Trp, $X^3$ représente Leu ou Cys, $X^4$ représente Pro ou Glu, $X^5$ représente Gln ou Asp, $X^5$ représente Thr ou Pro, $X^7$ représente His ou Tyr, $X^5$ représente Ser, $X^5$ représente Leu, $X^{10}$ représente Gly ou Ala, $X^{11}$ représente Ser ou Ala, $X^{16}$ représente Leu ou Met, $X^{22}$ représente Arg, $X^{23}$ représente Lys, $X^{26}$ représente Leu, $X^{27}$ représente Phe, $X^{31}$ représente Lys, $X^{34}$ représente His, $X^{38}$ représente Phe, $X^{43a}$

représente une liaison simple, $X^{50}$ représente Ala, $X^{51}$ représente Glu, $X^{52}$ représente Thr, $X^{54}$ représente Pro, $X^{53}$ représente Ile, $X^{58}$ représente Ser, $X^{70}$ représente Lys, $X^{77}$ représente Asp, $X^{78}$ représente Glu, $X^{79}$ représente Thr, $X^{52}$ représente Asp, $X^{55}$ représente Tyr, $X^{54}$ représente Asp, $X^{105}$ représente Val, Asp ou Thr, $X^{106}$ représente Thr, Leu ou Asn, $X^{120}$ représente Arg, $X^{124}$ représente Gln, $X^{131}$ représente Lys, $X^{151}$ représente Phe, $X^{153}$ représente Leu, $X^{156}$ représente Asn, $X^{157}$ représente Leu, $X^{158}$ représente Gln, $X^{159}$ représente Glu, $X^{160}$ représente Ser, $X^{161}$ représente Leu, $X^{162}$ représente Arg, $X^{163}$ représente Ser, $X^{164}$ représente Lys et $X^{165}$ représente Glu.

7. Polypeptide suivant la revendication 1, de formule :

$$
\begin{aligned}
&\text{Cys } X^2 X^3 X^4 X^5 X^6 X^7 \text{-Ser-Leu-} X^{10c}\text{-Ser-Arg-Arg-Thr-Leu-Met-} \\
&\text{Leu-Leu-Ala-Gln-Met-Arg-Lys-Ile-Ser-Leu-Phe-Ser-Cys-} \\
&\text{Leu-Lys-Asp-Arg-His-Asp-Phe-} X^{37}\text{-Phe-Pro-Gln-Glu-Glu-} \\
&\text{Phe-} X^{44}\text{-Asn-Gln-Phe-Gln-Lys-Ala-Glu-Thr-} \\
&\text{Ile-Pro-Val-Leu-His-Glu-} X^{59}\text{-Ile-Gln-Gln-Ile-Phe-Asn-} \\
&\text{Leu-Phe-Ser-Thr-Lys-Asp-Ser-Ser-Ala-Ala-Trp-Asp-Glu-} \\
&\text{Thr-Leu-Leu-Asp-Lys-Phe-Tyr-Thr-Glu-Leu-Tyr-Gln-Gln-} \\
&\text{Leu-Asn-Asp-Leu-Glu-Ala-} X^{98c}\text{-} X^{99c}\text{-} X^{100c}\text{-} X^{101c}\text{-} \\
&X^{102c}\text{-} X^{103c}\text{-} X^{104c}\text{-} X^{105c}\text{-} X^{106c}\text{-} X^{107c}\text{-} X^{108}\text{-} X^{109}\text{-} X^{110}\text{-} \\
&X^{111c}\text{-} X^{112c}\text{-} X^{113c}\text{-} X^{114}\text{-Ser-Ile-Leu-Ala-Val-Arg-Lys-} \\
&\text{Tyr-Phe-Gln-Arg-Ile-Thr-Leu-Tyr-Leu-Lys-Glu-Lys-Lys-} \\
&\text{Tyr-Ser-Pro-Cys-Ala-Trp-Glu-Val-Val-Arg-Ala-Glu-Ile-} \\
&\text{Met-Arg-Ser-Phe-Ser-Leu-Ser-Thr-Asn-Leu-Gln-Glu-Ser-} \\
&\text{Leu-Arg-Ser-Lys-Glu}
\end{aligned}
$$

(dans laquelle $X^2$ représente Asp ou Trp, $X^3$ représente Leu ou Cys, $X^4$ représente Pro ou Gln, $X^5$ représente Gln ou Asp, $X^5$ représente Thr ou Pro, $X^7$ représente His ou Tyr, $X^{10c}$ représente Gly ou Ala, $X^{37}$ représente Gly ou Ala, $X^{44}$ représente Gly ou Ala, $x^{59}$ représente Met ou Leu, $X^{98c}$ représente Cys ou Leu, $X^{99c}$ représente Val ou Thr, $X^{100c}$ représente Ile, Met ou Asn, $X^{101c}$ représente Gln ou Tyr, $X^{102c}$ représente Gly, Ala ou Ser, $X^{103c}$ représente Val, $X^{104c}$ représente Gly, Ala ou Thr, $X^{105c}$ représente Val ou Asp, $X^{106c}$ représente Thr ou Leu, $X^{107c}$ représente Glu ou Asn, $X^{108}$ représente Thr ou Val, $X^{109}$ représente Pro ou Gln, $X^{110}$ représente Leu ou Arg, $X^{111c}$ représente Met ou Lys, $X^{112c}$ représente Lys ou Ala, $X^{113c}$ représente Glu ou Ile, et $X^{114}$ représente Asp ou His) et polypeptides correspondants dans lesquels Cys en positions 29 et 138 est remplacé par Ser, les amino-acides étant choisis, de sorte que soit présent, au moins dans l'une des positions 2-7, 10, 37, 44, 59 et 99-114, un amino-acide qui diffère de l'amino-acide dans la position correspondante de IFN-alpha$_2$.

8. Polypeptide suivant la revendication 1, dans lequel $-X^{58}$-$X^{59}$-$X^{100}$-$X^{101}$-$X^{102}$-$X^{103}$-$X^{104}$-$X^{105}$-$X^{106}$-$X^{107}$-$X^{108}$-$X^{109}$-$X^{110}$-$X^{111}$-$X^{112}$-$X^{113}$-$X^{114}$- représente le groupement -Leu-Thr-Asn-Tyr-Ser-Val-Thr-Asp-Leu-Asn-Val-Gln-Arg-Lys-Ala-Ile-His.

9. Polypeptide suivant la revendication 1, dans lequel au moins l'un de $X^{10}$, $X^{37}$, $X^{44}$, $X^{102}$ et $X^{104}$ représente l'alanine.

49

**10.** Polypeptide suivant la revendication 1, dans lequel $X^{59}$ représente la leucine.

**11.** Polypeptide suivant la revendication 1, choisi dans le groupe comprenant :

```
IFN-alpha₂(4-155)
endo-Asp⁴³ᵃ-[Leu¹⁵¹,Arg¹⁶⁰,¹⁶³]IFN-alpha₂
[gamma(98-114)⁹⁸⁻¹¹⁴]IFN-alpha₂
[Met¹⁰⁰, gamma(101-114)¹⁰¹⁻¹¹⁴]IFN-alpha₂
[gamma(2-7), gamma(98-114)]IFN-alpha₂
[Leu⁵⁹]IFN-alpha₂
[Ala¹⁰,³⁷,⁴⁴,¹⁰²,¹⁰⁴]IFN-alpha₂
[Ser²⁹,¹³⁸]IFN-alpha₂
```

**12.** Procédé de production d'un polypeptide suivant la revendication 1, qui consiste à cultiver un micro-organisme, le micro-organisme ayant été transformé avec un vecteur d'expression plasmidique réplicable comprenant le matériau génétique codant pour ledit polypeptide, ce qui permet d'effectuer l'expression dudit polypeptide et de séparer ledit polypeptide ainsi exprimé.

**13.** Micro-organisme transformant capable d'effectuer l'expression d'un polypeptide suivant la revendication 1, ledit micro-organisme comprenant un vecteur d'expression plasmidique réplicable, vecteur qui comprend le matériel génétique codant pour ledit polypeptide.

**14.** Procédé de préparation d'un micro-organisme transformant suivant la revendication 13, qui consiste à transformer un micro-organisme par insertion dans ce microorganisme d'un vecteur d'expression plasmidique réplicable, vecteur qui comprend le matériel génétique codant pour un polypeptide suivant la revendication 1.

**15.** Vecteur d'expression plasmidique réplicable capable, dans un micro-organisme transformant, d'effectuer l'expression d'un polypeptide suivant la revendication 1.

**16.** Procédé de préparation d'un vecteur d'expression plasmidique réplicable suivant la revendication 15, qui consiste à insérer un gène codant pour un polypeptide suivant la revendication 1 dans un vecteur destiné à cette fin à un site d'insertion approprié.

**17.** Procédé de préparation d'un vecteur d'expression plasmidique réplicable suivant la revendication 15, qui consiste à insérer une séquence de nucléotides codant pour une portion du polypeptide suivant la revendication 1 dans un vecteur comprenant une séquence de promoteur et une ou plusieurs séquences de nucléotides codant pour le reste du polypeptide suivant la revendication 1 à un site d'insertion approprié, un vecteur d'expression plasmidique réplicable capable de diriger la synthèse dudit polypeptide dans un micro-organisme transformant étant ainsi obtenu.

**18.** Séquence d'ADN qui code pour un polypeptide suivant la revendication 1.

**19.** Unité d'expression mobile comprenant le segment EcoRI-SalI du plasmide pSTP1, dans lequel une séquence d'ADN suivant la revendication 18 est présente entre les sites de restriction ClaI et SalI dudit segment.

**20.** Composition pharmaceutique comprenant comme ingrédient actif au moins un polypeptide suivant la revendication 1, en association avec un support ou excipient pharmaceutiquement acceptable.

**Patentansprüche**

1. Ein Polypeptid der Formel I:

$$X^1-X^2-X^3-X^4-X^5-X^6-X^7-X^8-X^9-X^{10}-X^{11}-X^{12}-X^{13}-$$
$$-X^{14}-X^{15}-X^{16}-Leu-Leu-Ala-Gln-X^{21}-X^{22}-X^{23}-Ile-Ser-X^{26}-$$
$$-X^{27}-X^{28}-X^{29}-X^{30}-X^{31}-X^{32}-Arg-X^{34}-Asp-Phe-X^{37}-X^{38}-Pro-$$
$$-Gln-Glu-Glu-Phe-X^{43a}-X^{44}-Asn-Gln-Phe-Gln-Lys-X^{50}-X^{51}-$$
$$-X^{52}-Ile-X^{54}-Val-Leu-His-Glu-X^{59}-Ile-X^{61}-Gln-X^{63}-$$
$$-X^{64}-Asn-Leu-X^{67}-X^{68}-Thr-X^{70}-Asp-Ser-Ser-Ala-Ala-Trp-$$
$$-X^{77}-X^{78}-X^{79}-Leu-Leu-X^{82}-Lys-Phe-X^{85}-Thr-Glu-Leu-Tyr-$$
$$-Gln-Gln-Leu-Asn-X^{94}-Leu-Glu-Ala-X^{98}-X^{99}-X^{100}-X^{101}-$$
$$-X^{102}-X^{103}-X^{104}-X^{105}-X^{106}-X^{107}-X^{108}-X^{109}-X^{110}-X^{111}-$$
$$-X^{112}-X^{113}-X^{114}-X^{115}-Ile-Leu-Ala-Val-X^{120}-Lys-Tyr-Phe-$$
$$-X^{124}-Arg-Ile-Thr-Leu-Tyr-Leu-X^{131}-Glu-Lys-Lys-Tyr-$$
$$-Ser-X^{137}-X^{138}-Ala-Trp-Glu-Val-Val-Arg-Ala-Glu-$$
$$-Ile-X^{148}-Arg-Ser-X^{151}-Ser-X^{153}-Ser-X^{155}-X^{156}-X^{157}-$$
$$-X^{158}-X^{159}-X^{160}-X^{161}-X^{162}-X^{163}-X^{164}-X^{165}$$

in dem $X^1$, $X^2$, $X^3$, $X^4$, $X^5$, $X^5$, $X^7$, $X^5$, $X^5$, $X^{10}$, $X^{11}$, $X^{155}$, $X^{156}$, $X^{157}$, $X^{158}$, $X^{159}$, $X^{160}$, $X^{161}$, $X^{162}$, $X^{163}$, $X^{164}$ und $X^{165}$ jeweils eine natürlich vorkommende alpha-Aminosäure bedeuten können und $X^{12}$ Arg oder Ala bedeutet, $X^{13}$ Arg oder Ala bedeutet, $X^{14}$ Thr oder Ala bedeutet, $X^{15}$ Leu oder Ala bedeutet, $X^{16}$ Met, Leu oder Ile bedeutet, $X^{21}$ Met oder Leu bedeutet, $X^{22}$ Ser, Arg oder Gly bedeutet und $X^{23}$ Arg oder Lys bedeutet, $X^{26}$ Leu oder Pro bedeutet, $X^{27}$ Ser oder Phe bedeutet, $X^{28}$ Ser oder Ala bedeutet, $X^{29}$ Ser oder Cys bedeutet, $X^{30}$ Ile oder Leu bedeutet, $X^{31}$ Met oder Lys bedeutet, $X^{32}$ Ala, Asn, Asp oder Glu bedeutet und $X^{34}$ His oder Pro bedeutet, $X^{37}$ Gly oder Ala bedeutet, $X^{38}$ Phe oder Leu bedeutet, $X^{43a}$ eine Einfachbindung oder Asp bedeutet, $X^{44}$ Gly oder Ala bedeutet, $X^{50}$ Ala oder Thr bedeutet, $X^{51}$ Glu, Pro oder Gln bedeutet, $X^{52}$ Ala oder Thr bedeutet und $X^{54}$ Ser oder Pro bedeutet, $X^{59}$ Leu oder Met bedeutet, $X^{51}$ Gln oder Ala bedeutet, $X^{53}$ Ile oder Thr bedeutet, $X^{54}$ Phe oder Ala bedeutet, $X^{57}$ Phe oder Ala bedeutet, $X^{58}$ Thr oder Ser bedeutet, $X^{70}$ Lys oder Glu bedeutet, $X^{77}$ Asp oder Glu bedeutet, $X^{78}$ Glu oder Gln bedeutet, $X^{79}$ Asp, Thr oder Ser bedeutet, $X^{52}$ Asp oder Glu bedeutet, $X^{55}$ Cys, Tyr oder Ser bedeutet, $X^{54}$ Asp oder Asn bedeutet, $X^{58}$ Cys oder Leu bedeutet, $X^{59}$ Val oder Thr bedeutet, $X^{100}$ Met, Ile oder Asn bedeutet, und
entweder $X^{101}$ Gln, Tyr oder Phe bedeutet, $X^{102}$ Glu, Gly, Ser oder Ala bedeutet, $X^{103}$ Glu, Val oder Lys

bedeutet, $X^{104}$ Arg, Gly, Thr, Leu oder Ala bedeutet, $X^{105}$ Val, Asp, Met oder Thr bedeutet, $X^{106}$ Gly, Thr, Glu, Leu oder Asn bedeutet, $X^{107}$ Glu, Asn oder Tyr bedeutet, $X^{108}$ Thr oder Val bedeutet, $X^{109}$ Pro oder Gln bedeutet, $X^{110}$ Leu oder Arg bedeutet und $X^{111}$ Met, Lys oder Leu bedeutet,

oder $-X^{101}-X^{102}-X^{103}-X^{104}-X^{105}-X^{106}-X^{107}-X^{108}-X^{109}-X^{110}-X^{111}-$ Met, Lys oder Leu bedeutet, $-X^{112}$ Asn, Lys oder Ala bedeutet, $X^{113}$ Ala, Glu oder Ile bedeutet, $X^{114}$ Asp oder His bedeutet, $X^{115}$ Ser oder Phe bedeutet, $X^{120}$ Lys oder Arg bedeutet, $X^{124}$ Arg oder Gln bedeutet, $X^{131}$ Thr oder Lys bedeutet, $X^{137}$ Pro oder Ala bedeutet, $X^{138}$ Cys oder Ser bedeutet, $X^{148}$ Met oder Leu bedeutet, $X^{151}$ Leu oder Phe bedeutet und $X^{153}$ Leu oder Phe bedeutet, wobei die Aminosäuren so ausgewählt sind, daß eine Aminosäure wenigstens in einer Stellung vorhanden ist, die ausgewählt ist aus den alpha$_2$-Stellungen 12-16, 21, 28, 29, 30, 32, 37, 44, 61, 64, 67, 98-115, 137, 138 und 148, die sich von der Aminosäure in der entsprechenden Stellung von IFN-alpha$_2$, IFN-alpha$_1$ und IFN-alphaI unterscheidet, und/oder so ausgewählt sind, daß eine Aminosäure in wenigstens einer Stellung fehlt, die ausgewählt ist aus den alpha$_2$-Stellungen 101-110;

oder wobei die Aminosäuren so ausgewählt sind, daß eine Aminosäure in wenigstens einer Stellung vorhanden ist, die ausgewählt ist aus den alpha$_2$-Stellungen 27, 31, 59, 151, 160 und 163, die die gleiche ist wie die Aminosäure in der entsprechenden Stellung von IFN-alpha$_1$, wobei die Aminosäuren in den alpha$_2$-Stellungen, die nicht die Stellungen 1 bis 11, 27, 31, 43a, 59, 100, 102-104, 106, 112, 113, 151 und 156 bis 165 sind, die gleichen sind wie die Aminosäuren in den entsprechenden Stellungen von IFN-alpha$_2$ oder ein $N_{1-11}$ und/oder $C_{1-11}$-gekürztes Analoges davon und die $N_{3-11}$-gekürzten Analogen von IFN-alpha$_2$, die gewünschtenfalls $C_{1-11}$-gekürzt sein können, mit der Maßgabe, daß ein $N_3$-gekürztes Analoges von IFN-alpha$_2$ gleichzeitig $C_{7-11}$-gekürzt ist.

2. Ein Polypeptid nach Anspruch 1, worin $X^1$ Cys bedeutet, $X^2$ Asp oder Trp bedeutet, $X^3$ Leu oder Cys bedeutet, $X^4$ Pro oder Gln bedeutet, $X^5$ Glu, Gln oder Asp bedeutet, $X^5$ Thr oder Pro bedeutet, $X^7$ His oder Tyr bedeutet, $X^5$ Ser bedeutet, $X^5$ Leu bedeutet, $X^{10}$ Asp, Gly oder Ala bedeutet, $X^{11}$ Asn, Ser oder Ala bedeutet, $X^{155}$ Thr bedeutet, $X^{156}$ Asn bedeutet, $X^{157}$ Leu bedeutet, $X^{158}$ Gln bedeutet, $X^{159}$ Glu oder Lys bedeutet, $X^{160}$ Arg, Ser oder Ile bedeutet, $X^{161}$ Leu bedeutet, $X^{162}$ Arg bedeutet, $X^{163}$ Arg oder Ser bedeutet, $X^{164}$ Lys bedeutet und $X^{165}$ Glu oder Asp bedeutet.

3. Ein Polypeptid nach Anspruch 1, das $N_{1-3}$- und/oder $C_{1-10}$-gekürzt ist.

4.  Ein Polypeptid nach Anspruch 1 der Formel II:

```
Cys-Asp-Leu-Pro-X⁵ᵃ-Thr-His-Ser-Leu-X¹⁰ᵃ-X¹¹ᵃ-Arg-Arg-
  Thr-Leu-Met-Leu-Leu-Ala-Gln-Met-Arg-Lys-Ile-Ser-Leu-
  X²⁷ᵃ-Ser-Cys-Leu-X³¹ᵃ-Asp-Arg-His-Asp-Phe-Gly-Phe-Pro-
  Gln-Glu-Glu-Phe-X⁴³ᵃ-Gly-Asn-Gln-Phe-Gln-Lys-Ala-Glu-
  Tyr-Ile-Pro-Val-Leu-His-Glu-X⁵⁹ᵃ-Ile-Gln-Gln-Ile-Phe-
  Asn-Leu-Phe-Ser-Thr-Lys-Asp-Ser-Ser-Ala-Ala-Trp-Asp-
  Glu-Thr-Leu-Leu-Asp-Lys-Phe-Tyr-Thr-Glu-Leu-Tyr-Gln-
  Gln-Leu-Asn-Asp-Leu-Glu-Ala-Cys-Val-X¹⁰⁰ᵃ-Gln-
  X¹⁰²ᵃ-X¹⁰³ᵃ-X¹⁰⁴ᵃ-Val-X¹⁰⁶ᵃ-Glu- Thr-Pro-Leu-Met-
  X¹¹²ᵃ-X¹¹³ᵃ-Asp-Ser-Ile-Leu-Ala-Val-Arg-Lys-Tyr-Phe-
  Gln-Arg-Ile-Thr-Leu-Tyr-Leu-Lys-Glu-Lys-Lys-Tyr-Ser-
  Pro-Cys-Ala-Trp-Glu-Val-Val-Arg-Ala-Gln-Ile-Met-Arg-
  Ser-X¹⁵¹ᵃ-Ser-Leu-Ser-Thr-Asn-Leu-Gln-Glu-X¹⁶⁰ᵃ-
  Leu-Arg-X¹⁶³ᵃ-Lys-Glu
```

(worin X⁵ᵃ Glu oder Gln bedeutet, X¹⁰ᵃ Asp oder Gly bedeutet, X¹¹ᵃ Asn oder Ser bedeutet, X²⁷ᵃ Phe oder Ser bedeutet, X²¹ᵃ Lys oder Met bedeutet, X⁴³ᵃ Asp oder eine Einfachbindung bedeutet, X⁵⁹ᵃ Met oder Leu bedeutet, X¹⁰⁰ᵃ Met oder Ile bedeutet, X¹⁰²ᵃ Glu oder Gly bedeutet, X¹⁰³ᵃ Glu oder Val bedeutet, X¹⁰⁴ᵃ Arg oder Gly bedeutet, X¹⁰⁶ᵃ Gly oder Thr bedeutet, X¹¹²ᵃ Asn oder Lys bedeutet, X¹¹³ᵃ Ala oder Glu bedeutet, X¹⁵¹ᵃ Leu oder Phe bedeutet, X¹⁶⁰ᵃ Arg oder Ser bedeutet und X¹⁶³ᵃ Arg oder Ser bedeutet, mit der Maßgabe, daß die Aminosäuren so ausgewählt sind, daß wenigstens eine von X²⁷ᵃ, X²¹ᵃ, X⁵⁹ᵃ, X¹⁵¹ᵃ, X¹⁶⁰ᵃ und X¹⁶³ᵃ die gleiche Aminosäure bedeutet, wie die in der entsprechenden Stellung von IFN-alpha₁).

5.  Ein Polypeptid nach Anspruch 1 der Formel II, wie sie in Anspruch 4 definiert wird, in der X⁴³ᵃ Asp bedeutet, X¹⁰⁰ᵃ Ile bedeutet, X¹⁰²ᵃ Gly bedeutet, X¹⁰³ᵃ Val bedeutet, X¹⁰⁴ᵃ Gly bedeutet, X¹⁰⁶ᵃ Thr bedeutet, X¹¹²ᵃ Lys bedeutet und X¹¹³ᵃ Glu bedeutet, mit der Maßgabe, daß wenigstens eine von X⁵ᵃ, X¹⁰ᵃ, X¹¹ᵃ, X¹⁵¹ᵃ, X¹⁶⁰ᵃ und X¹⁶³ᵃ eine Aminosäure bedeutet, die sich von der Aminosäure in der entsprechenden Stellung von IFN-alpha₂ unterscheidet.

**6.** Ein Polypeptid nach Anspruch 1, worin $X^1$ Cys bedeutet, $X^2$ Asp oder Trp bedeutet, $X^3$ Leu oder Cys bedeutet, $X^4$ Pro oder Glu bedeutet, $X^5$ Gln oder Asp bedeutet, $X^5$ Thr oder Pro bedeutet, $X^7$ His oder Tyr bedeutet, $X^5$ Ser bedeutet, $X^5$ Leu bedeutet, $X^{10}$ Gly oder Ala bedeutet, $X^{11}$ Ser oder Ala bedeutet, $X^{16}$ Leu oder Met bedeutet, $X^{22}$ Arg bedeutet, $X^{23}$ Lys bedeutet, $X^{26}$ Leu bedeutet, $X^{27}$ Phe bedeutet, $X^{31}$ Lys bedeutet, $X^{34}$ His bedeutet, $X^{38}$ Phe bedeutet, $X^{43a}$ eine Einfachbindung bedeutet, $X^{50}$ Ala bedeutet, $X^{51}$ Glu bedeutet, $X^{52}$ Thr bedeutet, $X^{54}$ Pro bedeutet, $X^{53}$ Ile bedeutet, $X^{58}$ Ser bedeutet, $X^{70}$ Lys bedeutet, $X^{77}$ Asp bedeutet, $X^{78}$ Glu bedeutet, $X^{79}$ Thr bedeutet, $X^{52}$ Asp bedeutet, $X^{55}$ Tyr bedeutet, $X^{54}$ Asp bedeutet, $X^{105}$ Val, Asp oder Thr bedeutet, $X^{106}$ Thr, Leu oder Asn bedeutet, $X^{120}$ Arg bedeutet, $X^{124}$ Gln bedeutet, $X^{131}$ Lys bedeutet, $X^{151}$ Phe bedeutet, $X^{153}$ Leu bedeutet, $X^{156}$ Asn bedeutet, $X^{157}$ Leu bedeutet, $X^{158}$ Gln bedeutet, $X^{159}$ Glu bedeutet, $X^{160}$ Ser bedeutet, $X^{161}$ Leu bedeutet, $X^{162}$ Arg bedeutet, $X^{163}$ Ser bedeutet, $X^{164}$ Lys bedeutet und $X^{165}$ Glu bedeutet.

**7.** Ein Polypeptid nach Anspruch 1 der Formel:

$$\text{Cys } X^2 X^3 X^4 X^5 X^6 X^7 \text{-Ser-Leu-}X^{10c}\text{-Ser-Arg-Arg-Thr-Leu-Met-}$$
$$\text{Leu-Leu-Ala-Gln-Met-Arg-Lys-Ile-Ser-Leu-Phe-Ser-Cys-}$$
$$\text{Leu-Lys-Asp-Arg-His-Asp-Phe-}X^{37}\text{-Phe-Pro-Gln-Glu-Glu-}$$
$$\text{Phe-}X^{44}\text{-Asn-Gln-Phe-Gln-Lys-Ala-Glu-Thr-}$$
$$\text{Ile-Pro-Val-Leu-His-Glu-}X^{59}\text{-Ile-Gln-Gln-Ile-Phe-Asn-}$$
$$\text{Leu-Phe-Ser-Thr- Lys-Asp-Ser-Ser-Ala-Ala-Trp-Asp-Glu-}$$
$$\text{Thr-Leu-Leu-Asp-Lys-Phe-Tyr-Thr-Glu-Leu-Tyr-Gln-Gln-}$$
$$\text{Leu-Asn- Asp-Leu-Glu-Ala-}X^{98c}\text{-}X^{99c}\text{-}X^{100c}\text{-}X^{101c}$$
$$X^{102c}\text{-}X^{103c}\text{-}X^{104c}\text{-}X^{105c}\text{-}X^{106c}\text{-}X^{107c}\text{-}X^{108}\text{-}X^{109}\text{-}X^{110}$$
$$X^{111c}\text{-}X^{112c}\text{-}X^{113c}\text{-}X^{114}\text{-Ser-Ile-Leu-Ala-Val-Arg-Lys-}$$
$$\text{Tyr-Phe -Gln-Arg-Ile-Thr-Leu-Tyr-Leu-Lys-Glu-Lys-Lys-}$$
$$\text{Tyr-Ser-Pro-Cys-Ala-Trp-Glu-Val-Val-Arg-Ala-Glu-Ile-}$$
$$\text{Met- Arg-Ser-Phe-Ser-Leu-Ser-Thr-Asn-Leu-Gln-Glu-Ser-}$$
$$\text{Leu-Arg-Ser-Lys-Glu}$$

(worin $X^2$ Asp oder Trp bedeutet, $X^3$ Leu oder Cys bedeutet, $X^4$ Pro oder Gln bedeutet, $X^5$ Gln oder Asp bedeutet, $X^5$ Thr oder Pro bedeutet, $X^7$ His oder Tyr bedeutet, $X^{10c}$ Gly oder Ala bedeutet, $X^{37}$ Gly oder Ala bedeutet, $X^{44}$ Gly oder Ala bedeutet, $X^{59}$ Met oder Leu bedeutet, $X^{98c}$ Cys oder Leu bedeutet, $X^{99c}$ Val oder Thr bedeutet, $X^{100c}$ Ile, Met oder Asn bedeutet, $X^{101c}$ Gln oder Tyr bedeutet, $X^{102c}$ Gly, Ala oder Ser bedeutet, $X^{103c}$ Val bedeutet, $X^{104c}$ Gly, Ala oder Thr bedeutet, $X^{105c}$ Val oder Asp bedeutet, $K^{106c}$ Thr oder Leu bedeutet, $X^{107c}$ Glu oder Asn bedeutet, $X^{108}$ Thr oder Val bedeutet, $X^{109}$ Pro oder Gln bedeutet, $X^{110}$ Leu oder Arg bedeutet, $X^{111c}$ Met oder Lys bedeutet, $X^{112c}$ Lys oder Ala bedeutet, $X^{113c}$ Glu oder Ile bedeutet und $X^{114}$ Asp oder His bedeutet) und entsprechende Polypeptide, in denen Cys in den Stellungen 29 und 138 durch Ser ersetzt ist, wobei die Aminosäuren so ausgewählt sind, daß in wenigstens einer der Stellungen 2-7, 10, 37, 44, 59 und 99-114 eine Aminosäure vorhanden ist, die sich von der Aminosäure in der entsprechenden Stellung von IFN-alpha$_2$ unterscheidet.

**8.** Ein Polypeptid nach Anspruch 1, in dem -$X^{98}$-$X^{99}$-$X^{100}$-$X^{101}$-$X^{102}$-$X^{103}$-$X^{104}$-$X^{105}$-$X^{106}$-$X^{107}$-$X^{108}$-$X^{109}$-$X^{110}$-$X^{111}$-$X^{112}$-$X^{113}$-$X^{114}$- die Einheit -Leu-Thr-Asn-Tyr-Ser-Val-Thr-Asp-Leu-Asn-Val-Gln-Arg-Lys-Ala-Ile-His- bedeutet.

**9.** Ein Polypeptid nach Anspruch 1, in dem wenigstens eine von $X^{10}$, $X^{37}$, $X^{44}$, $X^{102}$ und $X^{104}$ Alanin bedeutet.

**10.** Ein Polypeptid nach Anspruch 1, in dem $X^{99}$ Leucin bedeutet.

**11.** Ein Polypeptid nach Anspruch 1, das ausgewählt ist aus der Gruppe, die besteht aus:

$$IFN\text{-}alpha_2(4\text{-}155)$$
$$endo\text{-}Asp^{43a}\text{-}[Leu^{151},Arg^{160,163}]IFN\text{-}alpha_2$$
$$[gamma(98\text{-}114)^{98\text{-}114}]IFN\text{-}alpha_2$$
$$[Met^{100}, gamma(101\text{-}114)^{101\text{-}114}]IFN\text{-}alpha_2$$
$$[gamma(2\text{-}7), gamma(98\text{-}114)]IFN\text{-}alpha_2$$
$$[Leu^{59}]IFN\text{-}alpha_2$$
$$[Ala^{10,37,44,102,104}]IFN\text{-}alpha_2$$
$$[Ser^{29,138}]IFN\text{-}alpha_2$$

**12.** Verfahren zur Herstellung eines Polypeptids nach Anspruch 1, das die Kultivierung eines Mikroorganismus umfaßt, wobei der Mikroorganismus mit einem replizierbaren plasmidischen Expressionsvehikel transformiert wurde, das ein genetisches Material enthielt, das für das genannte Polypeptid kodiert, um dadurch die Expression des genannten Polypeptids zu bewirken, und Gewinnen des auf diese Weise exprimierten genannten Polypeptids.

**13.** Ein transformierter Mikroorganismus, der in der Lage ist, ein Polypeptid nach Anspruch 1 zu exprimieren, wobei der genannte Mikroorganismus ein replizierbares plasmidisches Expressionsvehikel aufweist, wobei dieses Vehikel ein genetisches Material enthält, das für das genannte Polypeptid kodiert.

**14.** Verfahren zur Herstellung eines transformierten Mikroorganismus nach Anspruch 13, das das Transformieren eines Mikroorganismus durch Einführung eines replizierbaren plasmidischen Expressionsvehikels in diesen umfaßt, wobei dieses Vehikel ein genetisches Material enthält, das für ein Polypeptid nach Anspruch 1 kodiert.

**15.** Ein replizierbares plasmidisches Expressionsvehikel, das in der Lage ist, in einem transformierten Mikroorganismus ein Polypeptid nach Anspruch 1 zu exprimieren.

**16.** Verfahren zur Herstellung eines replizierbaren plasmidischen Expressionsvehikels nach Anspruch 15, das die Insertion eines Gens, das für ein Polypeptid nach Anspruch 1 kodiert, in einen Vektor dafür an einer geeigneten Insertionsstelle umfaßt.

**17.** Verfahren zur Herstellung eines replizierbaren plasmidischen Expressionsvehikels nach Anspruch 15, das die Insertion einer Nukleotidsequenz, die für einen Teil des Polypeptids nach Anspruch 1 kodiert, in einen Vektor mit einer Promotorsequenz sowie einer oder mehreren Nukleotidsequenz(en), die für den Rest des Polypeptids nach Anspruch 1 kodiert (kodieren), an einer geeigneten Insertionsstelle umfaßt, wodurch ein replizierbares plasmidisches Expressionsvehikel erhalten wird, das in der Lage ist, die Synthese des genannten Polypeptids in einem transformierten Mikroorganismus zu bewirken.

**18.** Eine DNA-Sequenz, die für ein Polypeptid nach Anspruch 1 kodiert.

**19.** Eine übertragbare Expressionseinheit mit dem EcoRI-Sall-Segment des Plasmids pSTP1, in der eine DNA-Sequenz nach Anspruch 18 zwischen den ClaI- und Sall-Restriktionsstellen des genannten Segments vorhanden ist.

**20.** Pharmazeutische Zusammensetzung, die als Wirkstoff wenigstens ein Polypeptid nach Anspruch 1 in Assoziierung mit einem pharmazeutisch zulässigen Träger oder Hilfsstoff enthält.

Figure 1 :

```
         1              A               10            B              20
     Met Cys Asp Leu Pro Gln Thr His Ser Leu Gly Ser Arg Arg Thr Leu Met Leu Leu Ala Gln Met Arg
      ←— 1 Taq —→  ←——— 3 ———→   ←——— 5 ———→      ←——— 7 ———→     ←——— 9 ———→
5'-CGACGAT GT GT GAT CT GCCGCAAACT CAT AGCCT GGGT AGCCGTCGCACCCT GAT GCT GCT GGCCCAAAT GCGC          -3'
3'-  TGCTACACACT AGACGGCGT TT GAGT AT CGGACGCAT CGGCAGCGT GGGACT ACGACGACCGGGT TT ACGCGGCAT AGAG -5'
         ←——— 2Taq —→   ←——— 4 ———→    ←——— 6 ———→     ←——— 8 ———→    ←——— 10 ———→

         30    C                                40            D
     Arg Ile Ser Leu Phe Ser Cys Leu Lys Asp Arg His Asp Phe Gly Phe Pro Gln Glu Glu Phe Gly Asn Gln Phe
     ←——— 11 ———→   ←——— 13 ———→    ←—— 15 ——→     ←——— 17 ———→    ←——— 19 ———→
5'-CGT ATCT CCCT GTT CT CCT GT CT GAAAGACCGCCAT GACTTT GGCTT CCCGCAGGAAGAGT T CGGT AACCAGTT C          -3'
3'-   GGACAAGAGGACAGACTTT CT GGCGGT ACTGAAACCGAAGGGCGT CCTT CT CAAGCCATT GGT CAAGGT TTT CCG -5'
         ←——— 12 ———→    ←——— 14 ———→    ←——— 16 ——→     ←——— 18 ———→    ←——— 20 ———→

         50              E              60                         F           70
     Gln Lys Ala Glu Thr Ile Pro Val Leu His Glu Met Ile Gln Gln Ile Phe Asn Leu Phe Ser Thr Lys Asp Ser
      ←—— 21 ——→    ←——— 23 ———→    ←—— 25 ——→     ←——— 27 ———→    ←——— 29 ———→
5'-CAAAAGGCAGAAACT AT CCCGGT ACT GCACGAAAT GAT T CAACAGAT TTTT AACCT GTT CAGCACT AAAGACT CC          -3'
3'-  TCT TTT GAT AGGGCCAT GACGT GCTT TACT AAGTT GT CT AAAAAATT GGACAAGT CGT GATTT CT GAGGAGAC GACG-5'
         ←——— 22 ———→    ←——— 24 ———→    ←—— 26 ——→     ←——— 28 ———→    ←——— 30 ———→

         80    G                        90            H
     Ser Ala Ala Trp Asp Glu Thr Leu Leu Asp Lys Phe Tyr Thr Glu Leu Tyr Gln Gln Leu Asn Asp Leu Glu Ala
      ←— 31 —→  ←— 33 —→    ←——— 35 ———→    ←——— 37 ———→    ←——— 39 ———→
5'-TCT GCT GCAT GGGACGAAACT CT CCT GGACAAATT CT ACACCGAACGT ACCAGCAACT GAACGACCT GGAAGCC          -3'
3'-  TACCCT GCTTT GAGAGGACCT GTTT AAGAT GT GGCTT GACAT GGT CGTT GACTT GCT GGACCTT CGGACGCAGT A-5'
         ←——— 32 ———→    ←——— 34 ———→    ←—— 36 ——→     ←——— 38 ———→    ←——— 40 ———→

         100             I              110           J    120
     Cys Val Ile Gln Gly Val Gly Val Thr Glu Thr Pro Leu Met Lys Glu Asp Ser Ile Leu Ala Val Arg Lys Tyr
      ←— 41 —→  ←— 43 —→    ←——— 45 ———→    ←——— 47 ———→    ←——— 49 ———→
5'-TGCGT CATCCAGGGT GTT GGCGT AACCGAAACT CCGCT GAT GAAAGAAGACT CCAT CCT GGCT GTT CGCAAAT AC          -3'
3'-   GGT CCCACAACCGCATT GGCTTT GAGGCGACT ACTTT CTT CT GAGGT AGGACCGACAAGCCGTTT AT GAAGGT CG-5'
         ←——— 42 ———→    ←——— 44 ———→    ←—— 46 ——→     ←——— 48 ———→    ←——— 50 ———→

         130   K                        140           L
     Phe Gln Arg Ile Thr Leu Tyr Leu Lys Glu Lys Lys Tyr Ser Pro Cys Ala Trp Glu Val Val Arg Ala Glu Ile
      ←— 51 —→  ←— 53 —→    ←——— 55 ———→    ←——— 57 ———→    ←——— 59 ———→
5'-TTCCAGCGT AT CACCCT GT ACCT GAAAGAGAAGAAAT ACAGCCCGT GCGCTT GGGAAGTT GT ACGCGCT GAAAT C          -3'
3'-  CAT AGT GGGACAT GGACTTT CT CTT CTTT AT GT CGGGCACGCGAACCCTT CAACAT GCGCGACTTT AGT ACGCAA-5'
         ←——— 52 ———→    ←——— 54 ———→    ←—— 56 ——→     ←——— 58 ———→    ←——— 60 ———→

         150             M    160           165
     Met Arg Ser Phe Ser Leu Ser Thr Asn Leu Gln Glu Ser Leu Arg Ser Lys Glu Stop  Sal I
      ←— 61 —→  ←—— 63 ——→    ←—— 65 ——→     ←——— 67 ———→
5'-ATGCGT T CCTT CAGCCT GT CCACT AACCT GCAAGAAT CT CT GCGT AGCCAAAGAAT AAG          -3'
3'-  GGAAGT CGGACAGGT GATT GGACGTT CTT AGAGACGCAT CGTTT CTT ATT CAGCT -5'
         ←——— 62 ———→    ←——— 64 ———→    ←—— 66 ——→     ←——— 68 ———→
```

57

FIG 2